# EUROPEAN PATENT APPLICATION

(11) **EP 4 159 845 A1**
(43) Date of publication of application: **05.04.2023**
(21) Application number: 20937456.0
(22) Date of filing: 22.07.2020
(51) Int. Cl.: C12N 5/09, C12N 5/071, C12Q 1/02, G01N 33/15

(54) **CULTURE MEDIUM FOR LARYNGEAL CANCER EPITHELIAL CELLS, CULTURE METHOD, AND APPLICATION THEREOF**

(30) Priority: 26.05.2020 CN 202010454162
(71) Applicant: CAS (Hefei) Institute of Technology Innovation, Hefei, Anhui 230088 (CN)
(72) Inventor: LIU, Qing Song, Hefei, Anhui 230031 (CN); HU, Jie, Hefei, Anhui 230031 (CN); REN, Tao, Hefei, Anhui 230031 (CN)
(74) Representative: Abel & Imray LLP
(86) International application number: PCT/CN2020/103428
(87) International publication number: WO 2021/237905

(57) **Abstract**

Provide are a primay cell culture medium that contains an MST1/2 kinase inhibitor and is used for culturing laryngeal cancer epithelial cells, and a culture method using the primary cell culture medium. In the culture method, the primay cell culture medium is used to culture primary cells on a clulture vessel plated with irradiated trophoblasts, so that the primay cells proliferate rapidly. A cell model obtained by the primary cell culture medium and the primay cell culture method can be used for the efficacy evaluation and screening of drugs.

## Description

### Technical Field

The invention relates to the field of medical technology, in particular to a culture medium and a culture method for culturing or expanding primary laryngeal cancer epithelial cells *in vitro,* and also to a method and use of the cultured cells in the efficacy evaluation and screening of drugs.

### Background of the Invention

The incidence rate of laryngeal cancer accounts for 5.7%-7.6% of malignant tumors in body. It ranks third only to nasopharyngeal carcinoma, and nasal cavity and paranasal sinus cancer in the field of otolaryngology. The prevalent age is 50 to 70. In China, the incidence rate is the highest in northeast, and the majority of the patients are males. The etiology is not very clear, but the cities with heavy air pollution have higher incidence rate than those with light pollution. Because the molecular pathogenesis of laryngeal cancer is still unclear, and the treatment methods for patients with laryngeal cancer, especially those in the middle and late stages, are still limited, resulting in a low five-year survival rate in clinical practice and lack of personalized and accurate drug guidance.

Functional testing refers to the method of detecting the sensitivity of antitumor drugs on cancer patient cells *in vitro.* The key to apply this method is to develop tumor cell models that have short growth cycle and can represent the biological characteristics of laryngeal cancer patients. In addition, the cell model should be easy to operate to quickly and efficiently predict the efficacy of clinical medication, so as to give precise medication guidance to cancer patients in a timely manner. However, the normally low success rates of *in vitro* establishment of cell models from the primary tumor cells of cancer patients, the long growth cycles, and the problems such as excessive proliferation of mesenchymal cells (such as fibroblasts, and the like), all restrict the development in this field. There are currently two technologies for culturing primary epithelial / stem cells that are relatively mature in the field of functional testing of tumor cells. One is the technology that uses irradiated trophoblastic cells and ROCK kinase inhibitor to promote the growth of primary epithelial cells to investigate the drug sensitivity of individual patients, that is, cell conditional reprogramming technology (Liu et al., Am J Pathol, 180: 599-607, 2012). Another technique is 3D culture of adult stem cells *in vitro* to obtain organoids similar to tissues and organs (Hans Clevers et al., Cell, 11; 172(1-2): 373-386, 2018).

The organoid technology is a technology that embeds the patient's autologous primary epithelial cells into the extracellular matrix for 3D *in vitro* culture; however, the culture medium of this technology needs to be added with a variety of specific growth factors (such as Wnt proteins and R-spondin family proteins), which is expensive and unsuitable for extensive use in clinical. In addition, the organoid needs to be embedded in the extracellular matrix gel during the entire culture process, and the plating steps of cell inoculation, passage, and drug sensitivity test are cumbersome and time-consuming as compared to 2D culture operations. Additionally, the size of the organoid formed by this technology is difficult to control, and some organoids may grow too large and cause an internal necrosis. Therefore, the organoid technology has poorer operability and applicability than the 2D culture technology. It requires professional technicians to operate, and thus, is not suitable for extensive and wide use in clinical for *in vitro* drug sensitivity testing (Nick Barker, Nat Cell Biol, 18(3): 246-54, 2016).

Cell reprogramming is a technique in which a patient's autologous primary epithelial cells are co-cultured with murine-derived feeder cells. However, it has not been reported in the literature to test the laryngeal cancer samples, so it is unknown whether the medium components reported in the literature can rapidly expand the primary laryngeal cancer cells.

In view of the limitations of the above technologies, it is necessary to develop a culture technology for primary laryngeal cancer epithelial cells in clinic, which may provide short culture period, controllable cost, and convenient operation. When the technology is applied to construct a primary tumor cell model of laryngeal cancer, the cultured laryngeal cancer cells can represent the biological characteristics of the laryngeal cancer patients. By evaluating *in vitro* the sensitivity of anti-tumor drugs in the cell models derived from individual cancer patients, the response rate of the anti-tumor drug can be improved in clinic, and the pains caused by inappropriate drugs to the patients and the wastes of medical resources can be reduced.

### Summary of the Invention

In view of the deficiencies of the prior art, the invention intends to provide a culture medium for culturing primary laryngeal cancer epithelial cells and a method for culturing primary laryngeal cancer epithelial cells using the culture medium. The culture medium and the culture method for primary laryngeal cancer epithelial cells of the invention can achieve the goal of short *in vitro* culture period, controllable cost, and convenient operation. When the technology is applied to construct a primary tumor cell model of laryngeal cancer, the primary laryngeal cancer cells with the biological characteristics of the laryngeal cancer patients can be obtained, which can be applied in new drug screening and *in vitro* drug sensitivity test.

One aspect of the invention is to provide a primary cell culture medium for culturing primary laryngeal cancer epithelial cells, comprising an MST1/2 kinase inhibitor, wherein the MST1/2 kinase inhibitor comprises a compound of Formula (I) or a pharmaceutically acceptable salt, or a solvate thereof. 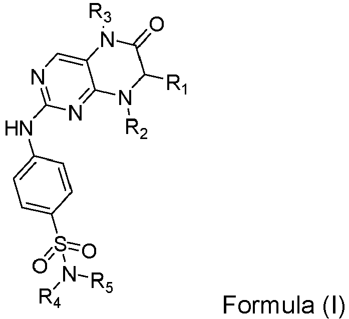 Wherein,
R₁ is selected from C1-C6 alkyl, C3-C6 cycloalkyl, C4-C8 cycloalkylalkyl, C2-C6 spirocycloalkyl, and aryl (e.g., phenyl and naphthyl, etc.) optionally independently substituted with 1-2 R₆, aryl C1-C6 alkyl (e.g., phenylmethyl, etc.) optionally independently substituted with 1-2 R₆ and heteroaryl (e.g., thienyl, etc.) optionally independently substituted with 1-2 R₆;
R₂ and R₃ are each independently selected from C1-C6 alkyl, preferably C1-C3 alkyl, more preferably methyl;
R₄ and R₅ are each independently selected from hydrogen, C1-C6 alkyl, C3-C6 cycloalkyl, C4-C8 cycloalkylalkyl, hydroxyl C1-C6 alkyl, C1-C6 haloalkyl, C1-C6 alkylamino C1-C6 alkyl, C1-C6 alkoxy C1-C6 alkyl, and C3-C6 heterocyclyl C1-C6 alkyl (the heterocyclyl is selected from e.g., piperidyl, tetrahydropyranyl, etc.);
R₆ is selected from halogen (preferably fluoro and chloro, more preferably fluoro), C1-C6 alkyl (preferably methyl), C1-C6 alkoxy (preferably methoxy), and C1-C6 haloalkyl (preferably trifluoromethyl).

In a preferable embodiment, the MST1/2 kinase inhibitor comprises a compound of Formula (Ia) or a pharmaceutically acceptable salt, or a solvate thereof, 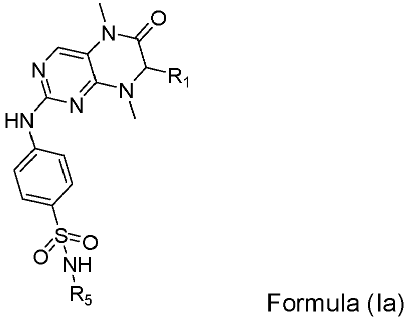 Wherein,
R₁ is selected from C1-C6 alkyl, phenyl optionally independently substituted with 1-2 R₆, thienyl optionally independently substituted with 1-2 R₆, and phenylmethyl optionally independently substituted with 1-2 R₆; more preferably, R₁ is phenyl optionally independently substituted with 1-2 R₆;
R₅ is selected from hydrogen, C1-C6 alkyl, and C3-C6 cycloalkyl; more preferably, R₅ is hydrogen;
R₆ is independently selected from halogen, C1-C6 alkyl, and C1-C6 haloalkyl; more preferably, R₆ is fluoro, methyl or trifluoromethyl.

Preferably, the MST1/2 kinase inhibitor is at least one selected from the following compounds or a pharmaceutically acceptable salt, or a solvate thereof.

| | | | |
|---|---|---|---|
| 1 | 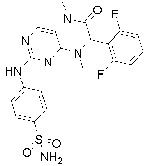 | 2 | 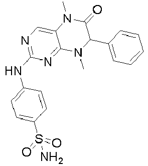 |
| 3 | 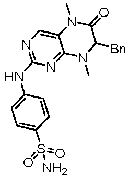 | 4 | 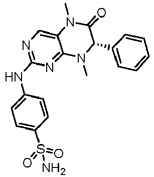 |
| 5 | 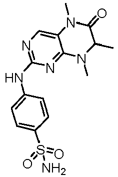 | 6 | 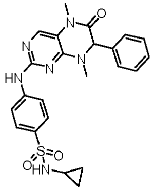 |
| 7 | 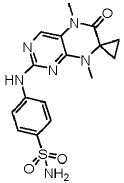 | 8 | 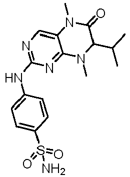 |
| 9 | 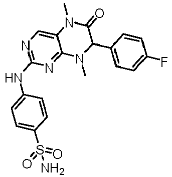 | 10 | 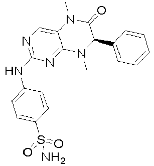 |
| 11 | 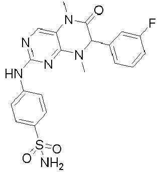 | 12 | 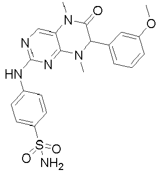 |
| 13 | 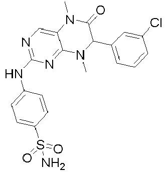 | 14 | 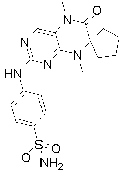 |
| 15 | 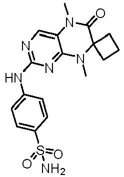 | 16 | 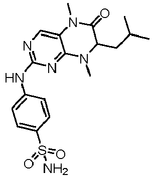 |
| 17 | 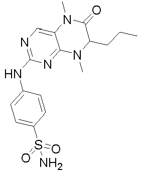 | 18 | 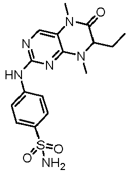 |
| 19 | 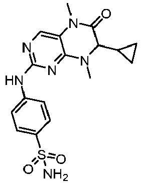 | 20 | 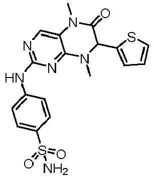 |
| 21 | 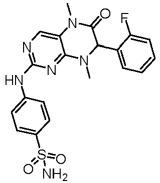 | 22 | 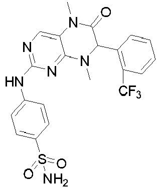 |
| 23 | 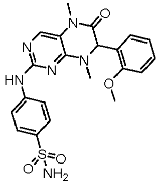 | 24 | 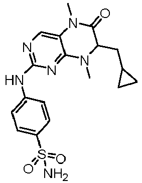 |
| 25 | 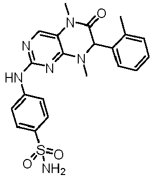 | 26 | 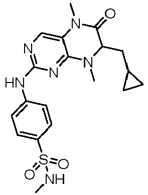 |
| 27 | 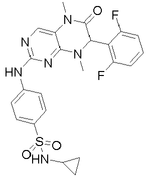 | 28 | 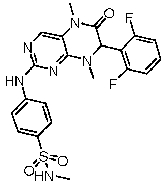 |
| 29 | 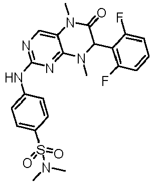 | 30 | 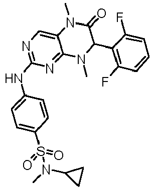 |
| 31 | 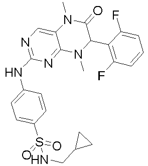 | 32 | 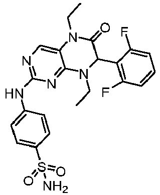 |
| 33 | 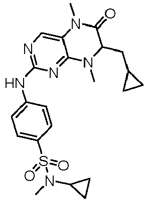 | 34 | 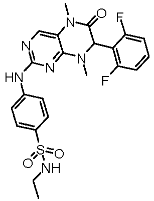 |
| 35 | 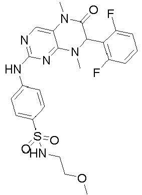 | 36 | 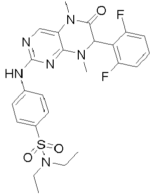 |
| 37 | 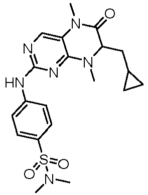 | 38 | 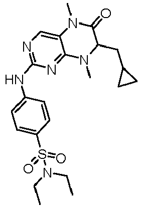 |
| 39 | 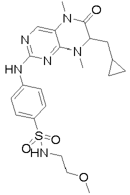 | 40 | 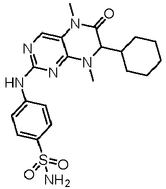 |
| 41 | 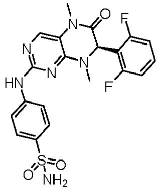 | 42 | 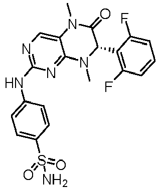 |
| 43 | 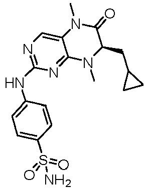 | 44 | 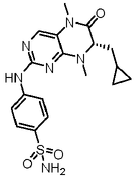 |
| 45 | 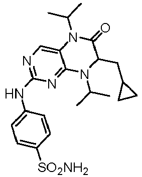 | 46 | 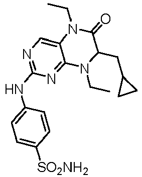 |
| 47 | 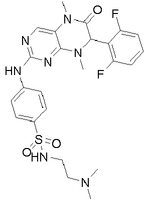 | 48 | 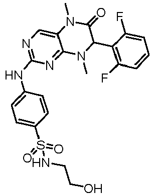 |
| 49 | 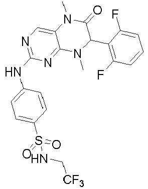 | 50 | 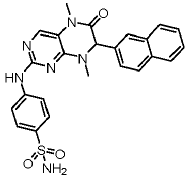 |
| 51 | 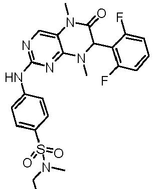 | 52 | 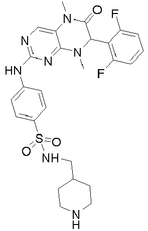 |
| 53 | 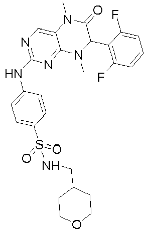 | 54 | 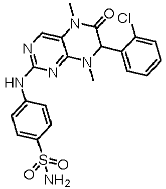 |
| 55 | 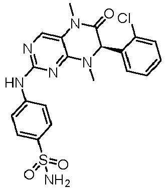 | 56 | 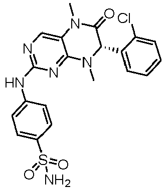 |
| 57 | 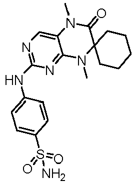 | 58 | 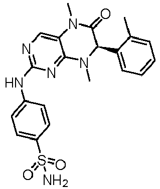 |
| 59 | 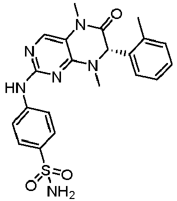 | | 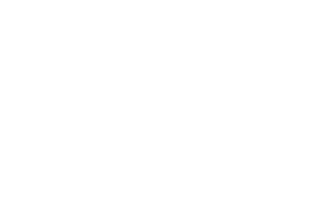 |

Most preferably, the MST1/2 kinase inhibitor of the invention is Compound 1.

In the embodiment of the invention, the amount of MST1/2 kinase inhibitor in the culture medium is usually 1.25 µM-10 µM, preferably 2.5 µM-10 µM, more preferably 5 µM.

Preferably, the primary cell culture medium of the invention further contains one or more of the following factors: insulin-like growth factor 1 (IGF-1); fibroblast growth factor 7 (FGF7); insulin-transferrin-selenium complex (ITS); hepatocyte growth factor (HGF); a ROCK kinase inhibitor selected from at least one of Y27632, Fasudil, and H-1152; and a TGFβ type I receptor inhibitor selected from at least one of A83-01, SB431542, Repsox, SB505124, SB525334, SD208, LY36494, and SJN2511.

In a preferred embodiment, the amount of fibroblast growth factor 7 in the culture medium is preferably 5-80 ng/ml, more preferably 20-80 ng/ml; the respective amounts of insulin / transferrin / sodium selenite in the insulin-transferrin-selenium complex are 5-20 µg/ml, 2.5-10 µg/ml, 2.5-10 ng/ml, respectively, and preferably 10-20 µg/ml, 5-10 µg/ml, 5-10 ng/ml, respectively; the amount of insulin-like growth factor 1 is preferably 10-80 ng/ml, more preferably 10-40 ng/ml; the amount of hepatocyte growth factor is preferably 10-80 ng/ml, more preferably 10-40 ng/ml; the ROCK kinase inhibitor is preferably Y27632, and the amount of the ROCK kinase inhibitor is preferably 1.25-20 µM, more preferably 2.5-10 µM; the TGFβ type I receptor inhibitor is preferably A83-01, and the amount of the TGFβ type I receptor inhibitor is preferably 125 -1000 nM, more preferably 125-500 nM.

Compared to the cell conditional reprogramming medium and the organoid medium, the composition of this medium is supplemented with an MST1/2 kinase inhibitor, but does not contain uncertain components such as serum, bovine pituitary extract or the like, niche factors that are necessary for organoid culture such as Wnt agonists, R-spondin family proteins, BMP inhibitors or the like, nor does it contain nicotinamide, and N-acetylcysteine, thereby greatly reducing the cost of the medium, simplifying the operation process of preparing the medium, and realizing the *in vitro* culture of the primary laryngeal cancer epithelial cells with controllable cost and convenient operation.

In the invention, the primary laryngeal cancer epithelial cells can be selected from laryngeal cancer cells, normal laryngeal cancer epithelial cells, and laryngeal cancer epithelial stem cells.

One aspect of the invention is to provide a method for culturing the primary laryngeal cancer epithelial cells, comprising the following steps:
(1) Preparing the primary cell culture medium of the invention according to the above composition.
(2) Pre-laying the culture container with irradiated trophoblastic cells.
   Specifically, the trophoblastic cells can be, for example, irradiated NIH-3T3 cells, and the irradiation source is X-ray or γ-ray, preferably γ-ray, with irradiation dose of 30-50 Gy, preferably 35 Gy. Specifically, the irradiated NIH-3T3 cells are inoculated into the culture container such as 48-well plate, 24-well plate, 12-well plate, 6-well plate or T25 cell culture flask at a density of 2×10⁴ cells/cm², and are reserved for use after being adhered to the wall.
(3) Isolating the primary laryngeal cancer epithelial cells from the laryngeal cancer tissue.

The primary laryngeal cancer epithelial cells can be derived, for example, from the laryngeal cancer tissue samples and paracancerous tissue samples. For example, the laryngeal cancer tissue samples are derived from the cancer tissue of the informed and consenting laryngeal cancer patient by surgical resection, and the paracancerous tissue samples are collected from the laryngeal cancer tissue at least 5 cm away from the laryngeal tissue. Collection of the aforementioned tissue samples is performed within half an hour of the surgical excision or the biopsy. More specifically, in a sterile environment, the tissue sample from non-necrotic sites is cut, with its volume of more than 0.5 cm³, and then the tissue sample is placed in pre-cooled 3-5 mL DMEM/F12 medium, which is contained in a plastic sterile centrifuge tube with a lid, and transported to the laboratory on ice. Wherein, the DMEM/F12 medium contains 1-2 vol% of penicillin/streptomycin, and/or 0.2-0.4 vol% of Primocin (hereinafter referred to as the tissue transport fluid). In case of using streptomycin/penicillin, the concentration range of streptomycin is 25-400 µg/mL, preferably 50-200 µg/mL, more preferably 200 µg/mL, the concentration range of penicillin is 25-400 U/mL, preferably 50-200 U/mL, more preferably 200 U/mL; and in case of using Primocin, the concentration range is 25-400 µg/mL, preferably 50-200 µg/mL, more preferably 100 µg/mL.

In a biological safety cabinet, the tissue sample is transferred to a cell culture dish, which is then rinsed with the transport fluid, and the blood cells on the surface of the tissue sample are washed away. The rinsed tissue sample is transferred to another new culture dish, with the addition of 1-3 mL of the transport fluid, and the tissue sample is divided into tissue fragments less than 3 mm³ in volume using a sterile scalpel blade and a forceps.

The tissue sample fragments are transferred to a centrifuge tube, which is centrifuged at 1000-3000 rpm for 3-5 minutes in a tabletop centrifuge (Sigma, 3-18K); after discarding the supernatant, the tissue transport fluid and the tissue digestive solution are added in a ratio of 1:1 (the dosage is about 5 mL of tissue digestive solution per 10 mg of tissue; the preparation method of the tissue digestive solution comprises: dissolving 1-2 mg/mL collagenase II, 1-2 mg/mL collagenase IV, 50-100 U/mL deoxyribonucleic acid I, 0.5-1 mg/mL hyaluronidase, 0.1-0.5 mg/mL calcium chloride, 5-10 mg/mL bovine serum albumin in HBSS and RPMI-1640 with a volume ratio of 1:1); then the sample is numbered and sealed with sealing film, and is then digested in a constant-temperature shaker (Zhichu Instrument ZQLY-180N) at 37°C, 200-300 revolutions; whether the digestion is completed is determined via observation every 1 hour; if no obvious tissue block is found, the digestion can be terminated; otherwise, it is continued for digestion until the digestion is sufficient, with the digestion time ranges from 4 to 8 hours. After digestion, undigested tissue blocks are filtered through a cell filter screen (the cell screen mesh size is, for example 70 µm); the tissue blocks on the filter screen are rinsed with the tissue transport fluid; the residual cells are rinsed into a centrifuge tube and centrifuged with a tabletop centrifuge at 1000-3000 rpm for 3-5 minutes. After discarding the supernatant, the remaining cell clusters are observed to determine whether blood cells are remained; if there are blood cells, 1-5 mL blood cell lysate (purchased from Sigma) is added, which is then mixed well, lysed at 4°C for 10-20 minutes, with shaking and mixing well once every 5 minutes; after lysis, the resultant is take out and centrifuged at 1000-3000 rpm for 3-5 minutes. After discarding the supernatant, the primary cell culture medium of the invention is added for resuspension. The total number of cells is obtained by counting with a flow imaging counter (JIMBIO FIL, Jiangsu Jimbio Technology Co., Ltd.).

(4) Inoculating the primary laryngeal cancer epithelial cells isolated in step (3) in the culture vessel that pre-inoculated with trophoblastic cells, and culturing by using the primary cell culture medium obtained in step (1).

More specifically, NIH-3T3 cells that are irradiated with γ-ray with irradiation dose of 35 Gy, are pre-inocluated at a density of 2×10⁴ to 4×10⁴ cells/cm² (e.g., 2 × 10⁴ cells/cm²) in one well of a multi-well plate, and after being adhered to the wall, primary laryngeal cancer cells are inoculated at a density of 2×10⁴ to 8×10⁴ cells/cm² (e.g., 4 × 10⁴ cells/cm²); 0.5-2 mL of primary epithelial cell culture medium is added to each well, and then the plate is cultured in a cell incubator for 8-16 days, for example, under the conditions of 37°C, 5% CO₂; fresh primary cell culture medium is used for replacing every 4 days during the culture; digestion and passaging are performed when the primary laryngeal cancer epithelial cells grow to a cell density that accounts for about 80% to 90% of the bottom area of the multi-well plate.

Compared to the organoid technology, this step does not need to mix the primary cells with the matrix gel uniformly on ice to form gel droplets and wait for the solidification of the gel droplets before adding the medium. In addition, it saves the amount of the expensive extracellular matrix gel and simplifies the operation steps.

Optionally, after culturing the inoculated primary laryngeal cancer epithelial cells for 8-16 days, when the cell clones formed in the culture container converge to cover 80% of the bottom area, the supernatant is discarded, and 1-2 mL of 0.25% trypsin (purchased from Thermo Fisher) is added for digestion for 1 minute; after removing 0.25% trypsin, 1-2 mL of 0.05% trypsin is added again for cell digestion, which is then incubated at room temperature for 5-20 min. The digested cells are resuspended in 2-4 mL of culture liquid containing, for example, 5% (v/v) fetal bovine serum, 100 U/mL penicillin, and 100 µg/mL streptomycin, and are centrifuged at 1000-3000 rpm for 3-5 minutes. The digested single cells are resuspended using the primary cell culture medium of the invention, and the obtained cell suspension is placed in a T25 cell culture flask pre-laid with trophoblastic cells for continuous culture. The pretreatment operation of T25 cell culture flask is the same as that in step (2).

The expanded laryngeal cancer epithelial cells grow in 2D, avoiding the non-uniform size of organoids and internal necrosis in overgrown organoids that may occur in the expansion using organoid technology.

In another aspact, the laryngeal cancer epithelial cells, especially the laryngeal cancer cells, cultured by the culturing method for primary laryngeal cancer epithelial cells of the invention, can be used for drug efficacy evaluation and drug screening, which comprises the following steps:
(1) Obtaining the primary laryngeal cancer epithelial cells, more preferably, obtaining the cancer tissue samples or the biopsy cancer tissue samples derived from the laryngeal cancer patients; isolating the primary laryngeal cancer epithelial cells, and culturing and expanding the primary laryngeal cancer epithelial cells (particularly the primary laryngeal cancer cells) according to the method for culturing the primary laryngeal cancer epithelial cells described above to a cell number of at least the magnitudes of 10⁵, preferably at least the magnitudes of 10⁶.
(2) Selecting the drug for testing.
(3) Based on the maximum plasma concentration of the drug Cₘₐₓ as a reference, taking 2-5 times of Cₘₐₓ as the initial concentration, and diluting the drug into different drug concentration gradients, such as 5-10 drug concentration gradients, preferably 6-8 drug concentration gradients.
(4) Digesting the laryngeal cancer epithelium cells cultured in step (1) into a single cell suspension, counting the cell number with a flow imaging counter, diluting the single cell suspension with the primary cell culture medium of the invention, adding the diluted cell suspension evenly to the multi-well plate at a density of 2000-4000 cells per well, e.g., 50 µL of cell dilution per well, and adhering overnight.
   This step avoids the problem of the cell reprogramming technique that the presence of feeder cells may interfere with the primary cell counting and the subsequent primary cell viability assay, and eliminates the need of the tedious step of mixing, embedding and then plating the cell suspension with matrix gel on ice as that in the organoid technique, which greatly simplifies the operation process and enhances the operability and practicality of the technology. Since the inoculated cells are single-cell suspensions rather than 3D structures like organoid, this technique may result in a more uniform number of plating cells and less variation in cell numbers between wells when compared to the organoid technique, making it more suitable for subsequent high-throughput drug screening operations.
(5) Adding the selected candidate drugs such as traditional chemotherapeutic drugs, targeted drugs, antibody drugs, or combination thereof with gradient dilutions, to the adherent cells obtained in step (4), using a high-throughput automated workstation.
(6) After a few hours of drug addition, for example, 72 hours, detecting the survival rate of the laryngeal cancer epithelial cells using the Cell-Titer Glo luminescence cell viability detection kit (purchased from Promega) to screen for drug activity.

Specifically, each well is added with, for example, 10 µL of Cell Titer-Glo reagent (purchased from Promega), and after uniformly shaking, the chemiluminescence intensity of each well is measured with a fluorescence microplate reader. Taking the drug concentration as the abscissa and the fluorescence intensity as the ordinate, GraphPad Prism software is used to draw the drug dose-effect curve based on the measured values, and the inhibitory intensity of the drugs on the proliferation of the tested cells are calculated.

When using the primary laryngeal cancer cells of the invention in drug screening and *in vitro* drug sensitivity test, due to the 2D growth of the cells, the interaction with the drug is quicker than the drug test time in the organoid technology (the average administration time in the organoid technology is 6 days).

The beneficial effects of the invention also include:
(1) the success rate for culturing the primary laryngeal cancer epithelial cell can be improved, with a success rate of more than 85%;
(2) the laryngeal cancer epithelial cells primary cultured *in vitro* can be ensured to reproduce the pathological phenotype and the heterogeneity of the owner patient of the primary cells;
(3) the composition of the medium does not contain serum, and thus, it is not affected by the quality and the quantity of the serum from different batches;
(4) the laryngeal cancer epithelial cells can be expanded with high efficiency, with a magnitude of 10⁶ of laryngeal cancer epithelial cells being successfully expanded within about two weeks from the starting of 10⁴-level cell count, and the expanded laryngeal cancer epithelial cells have the ability of continuous passage;
(5) there is no need to operate on ice and to dissociate the matrix gel in the passage step, and the digestion and passage of cells can be completed within 10-15 minutes;
(6) the cost for culture is controllable, as the primary laryngeal cancer culture medium does not require expensive Wnt agonists, R-spondin family proteins, BMP inhibitors and the like factors, and the cost for cell culture can be saved;
(7) the operations are convenient: compared with the organoid technology, there is no need to embed cells in the matrix gel as in the organoid technology, and the operation steps are simple and easy;
(9) this technology can culture and provide the laryngeal cancer epithelial cells with large quantity and high uniformity, which is suitable for high-throughput screening of new candidate compounds and high-throughput drug sensitivity functional tests *in vitro* for patients.

Using the cell culture medium of this embodiment, the laryngeal cancer epithelial cells derived from humans or other mammals, including laryngeal cancer cells, normal laryngeal cancer epithelial cells, laryngeal cancer epithelial stem cells, or tissues comprising at least any of these cells, can be cultured. At the same time, the culture medium of the invention can also be used to develop a kit for expansion and culture of primary laryngeal cancer cells *in vitro.*

Furthermore, the cells obtained by the culture method of this embodiment can be used in regenerative medicine, basic medical research of laryngeal cancer epithelial cells, screening of drug responses, and development of new drugs for ESCC diseases, and the like.

### Description of Drawings

Figs. 1A-1F show the effects of different factors in the culture medium on the proliferation of primary laryngeal cancer cells.
Fig. 2 shows the effect of increasing factors in the culture medium on the proliferation of primary laryngeal cancer cells.
Figs. 3A-3G show the effect of the concentration of each factor on the proliferation of primary laryngeal cancer cells.
Figs. 4A and 4B are photographs of laryngeal cancer cells taken under an inverted microscope, which were cultured from the cells isolated from one clinical laryngeal cancer tissue sample (No. 0S0003), by using the culture medium SCM of the invention, to Day 4 and Day 12, respectively.
Fig. 5 is a comparative view of the total number of cells, which were collected after 7 days of culture in 6 different culture mediums from the cells isolated from one surgically resected sample of laryngeal cancer (No. 0S0006).
Fig. 6 is a comparative diagram of cell proliferation effects obtained by culturing the cells isolated from six surgically resected samples of laryngeal cancer (No. 0S0011, 0S0012, 0S0013, 0S0014, 0S0015, 0S0016) for 7 days under the conditions of 6 different culture mediums.
Fig. 7 is a comparative diagram of cell growth curves obtained by culturing the cells isolated from one clinical laryngeal cancer tissue sample (No. 0S0004) under the conditions of 6 different culture mediums.
Figs. 8A and 8B are pictures of the laryngeal cancer cells, which were obtained by culturing the cells isolated from one surgically resected sample of laryngeal cancer (No. 0S0015) with the culture medium SCM of the invention, and then were stained with non-specific nuclear dye DAPI and laryngeal cancer specific antibody p63, respectively.
Figure 9 is a comparison of immunohistochemical results of the original tissue cells of one surgically resected sample of laryngeal cancer (No. 0S0001) and the laryngeal cancer cells obtained by culturing the cells with the culture medium SCM of the invention.
Figs. 10A and 10B show the dose-response curves of laryngeal cancer cells on different chemotherapeutic drugs and targeted drugs, wherein the laryngeal cancer cells were obtained by culturing the surgically resected cancer tissue samples of two different laryngeal cancer patients (No. 0S0020 and No. 0S0022) with the culture medium SCM of the invention, respectively.

### Detailed Description of the Invention

In the specification, the epithelial cells include differentiated epithelial cells and epithelial stem cells obtained from epithelial tissues. "Epithelial stem cells" refer to the cells with long-term self-renewal ability that may differentiate into epithelial cells, and the stem cells originated from epithelial tissues. Examples of epithelial tissues include cornea, oral mucosa, skin, conjunctiva, bladder, renal tubule, kidney, digestive organs (esophagus, stomach, duodenum, small intestine (including jejunum and ileum), large intestine (including colon)), liver, pancreas, mammary gland, salivary gland, lacrimal gland, prostate, hair root, trachea, lung, etc. Among others, the cell culture medium of the embodiment is preferably the culture medium for laryngeal cancer originated epithelial cells.

In addition, in this specification, "epithelial tumor cells" refer to the cells obtained by tumorigenesis of cells originated from the aforementioned epithelial tissues.

In the specification, "organoid" refers to a three-dimensional, organ-like cellular tissue formed by spontaneously organizing and aggregating cells within a controlled space in high density.

### [Preparation Examples of MST1/2 Kinase Inhibitors]

In the specification, MST1/2 kinase inhibitor refers to any inhibitor that directly or indirectly negatively regulates MST1/2 signaling. Generally, MST1/2 kinase inhibitors reduce the activity of MST1/2 kinase by, for example, binding to the same. Since MST1 and MST2 have similar structures, MST1/2 kinase inhibitors may be, for example, compounds that bind to MST1 or MST2 and reduce the activity thereof.

### 1. Preparation of MST1/2 kinase inhibitor Compound 1

### 4-((7-(2,6-difluorophenyl)-5,8-dimethyl-6-oxo-5,6,7,8-tetrahydropteridin-2-yl) amino)benzsulfamide 1

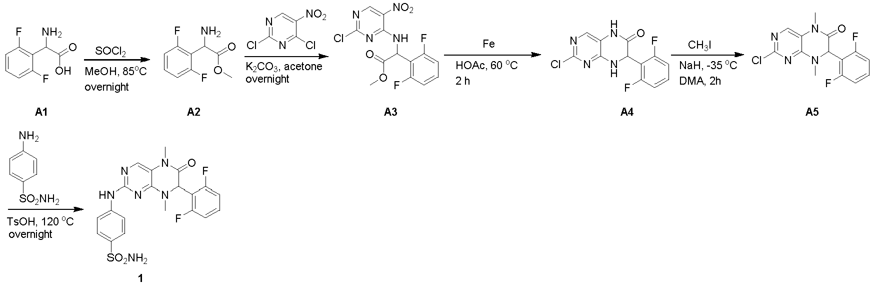

Methyl 2-amino-2-(2,6-difluorophenyl)acetate (A2): 2-amino-2-(2,6-difluorophenyl)acetic acid (2.0 g) and then methanol (30 ml) were added into a round bottom flask, followed by addition of thionyl chloride (1.2 ml) dropwise under an ice bath. The reaction system was reacted overnight at 85°C. After the completion of the reaction, the system was evaporated under reduced pressure to dry the solvent, and the obtained white solid was directly used in the next step.

Methyl 2-((2-chloro-5-nitropyrimidin-4-yl)amino)-2-(2,6-difluorophenyl) acetate (A3): methyl 2-amino-2-(2,6-difluorophenyl)acetate (2 g) and then acetone (30 ml) and potassium carbonate (2.2 g) were added into a round bottom flask, and then the system was cooled to -10°C with an ice salt bath, and then a solution of 2,4-dichloro-5-nitropyrimidine (3.1 g) in acetone was slowly added. The reaction system was stirred overnight at room temperature. After the completion of the reaction, the reaction mixture was filtered, the solvent was removed from the filtrate under reduced pressure, and the residue was purified by pressurized silica gel column chromatography to obtain compound A3. LC/MS: M+H 359.0.

2-chloro-7-(2,6-difluorophenyl)-7,8-dihydropteridin-6(5H)-one (A4): methyl 2-((2-chloro-5-nitropyrimidin-4-yl)amino)-2-(2,6-difluorophenyl)acetate (2.5g) and then acetic acid (50 ml) and iron powder (3.9 g) were added into a round bottom flask. The reaction system was stirred at 60°C for two hours. After the completion of the reaction, the reaction system was evaporated under reduced pressure to dry the solvent, and the resultant was neutralized to be alkaline with saturated sodium bicarbonate solution and was extracted with ethyl acetate. The organic phase was washed with water and saturated brine and dried with anhydrous sodium sulfate. The organic phase was filtered and evaporated to dryness under reduced pressure to obtain a crude product. The crude product was washed with diethyl ether to obtain compound A4. LC/MS: M+H 297.0.

2-chloro-7-(2,6-difluorophenyl)-5,8-dimethyl-7,8-dihydropteridin-6(5H)-one (A5): 2-chloro-7-(2,6-difluorophenyl)-7,8-dihydropteridin-6(5H)-one (2 g) and N,N-dimethylacetamide (10 ml) were added into a round bottom flask, and cooled to -35°C, followed by addition of iodomethane (0.9 ml) and then sodium hydride (615 mg), and the reaction system was stirred for two hours. After the completion of the reaction, the reaction mixture was quenched with water, and extracted with ethyl acetate. The organic phase was washed with water and saturated brine, respectively, and dried with anhydrous sodium sulfate. The organic phase was filtered and evaporated to dryness under reduced pressure to obtain a crude product. The crude product was washed with diethyl ether to obtain compound A5. LC/MS: M+H 325.0.

4-((7-(2,6-difluorophenyl)-5,8-dimethyl-6-oxo-5,6,7,8-tetrahydropteridin-2-yl )amino)benzsulfamide (1): 2-chloro-7-(2,6-difluorophenyl)-5,8-dimethyl-7,8-dihydropteridin-6(5H)-one (100 mg), sulfanilamide (53 mg), p-toluenesulfonic acid (53 mg) and sec-butanol (5 ml) were added into a round bottom flask. The reaction system was stirred at 120°C overnight. After the completion of the reaction, the reaction mixture was filtered, and washed with methanol and diethyl ether to obtain compound 1. LC/MS: M+H 461.1.

### 2. Preparation of other MST1/2 inhibitor compounds of the invention

Other MST1/2 inhibitor compounds of the invention were synthesized via the method similar to that of Compound 1, and their structures and mass spectrum data are shown in the following table.

| No. | Compound | MS(ESI) m/z(M+1)+ | No. | Compound | MS(ESI) m/z(M+1)+ |
|---|---|---|---|---|---|
| 1 | 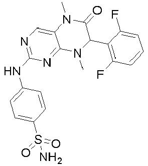 | 461.1 | 2 | 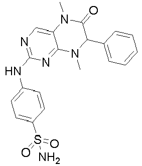 | 425.14 |
| 3 | 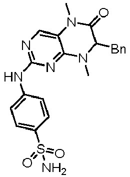 | 439.15 | 4 | 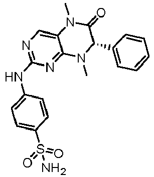 | 425.14 |
| 5 | 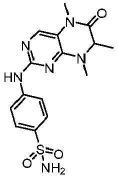 | 363.12 | 6 | 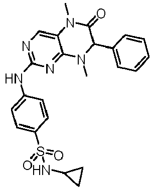 | 465.17 |
| 7 | 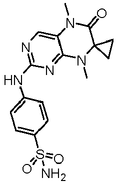 | 375.12 | 8 | 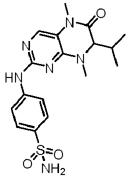 | 391.16 |
| 9 | 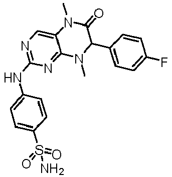 | 443.13 | 10 | 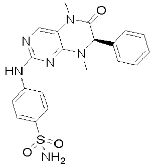 | 425.14 |
| 11 | 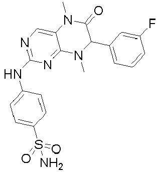 | 443.13 | 12 | 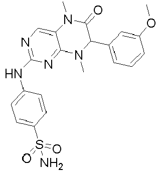 | 455.15 |
| 13 | 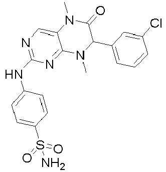 | 459.10 | 14 | 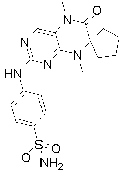 | 403.15 |
| 15 | 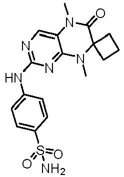 | 389.14 | 16 | 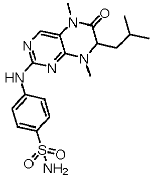 | 405.17 |
| 17 | 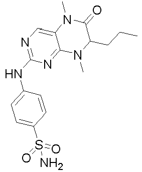 | 391.15 | 18 | 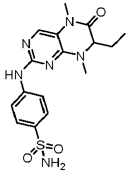 | 377.14 |
| 19 | 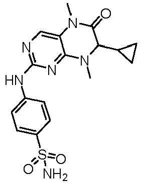 | 389.14 | 20 | 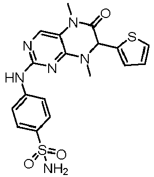 | 431.09 |
| 21 | 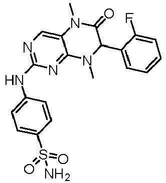 | 443.13 | 22 | 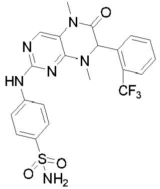 | 493.12 |
| 23 | 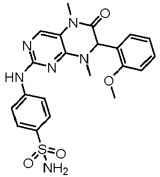 | 455.15 | 24 | 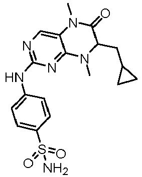 | 403.15 |
| 25 | 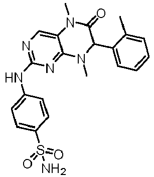 | 439.15 | 26 | 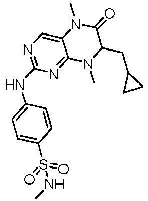 | 417.17 |
| 27 | 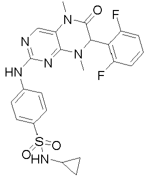 | 501.15 | 28 | 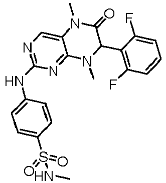 | 475.13 |
| 29 | 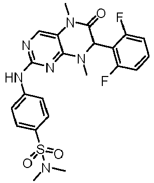 | 489.15 | 30 | 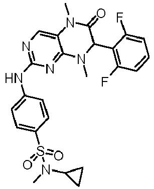 | 515.16 |
| 31 | 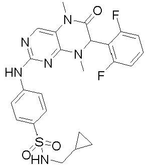 | 515.16 | 32 | 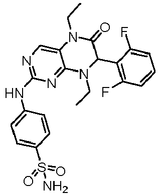 | 489.15 |
| 33 | 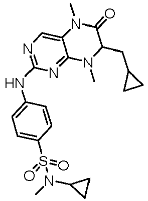 | 457.20 | 34 | 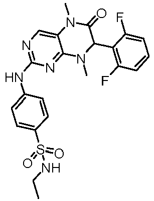 | 489.15 |
| 35 | 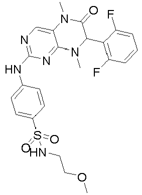 | 519.16 | 36 | 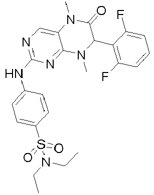 | 517.18 |
| 37 | 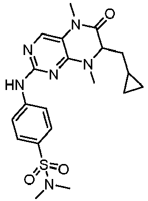 | 431.18 | 38 | 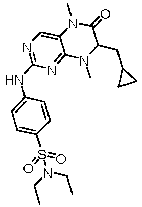 | 459.21 |
| 39 | 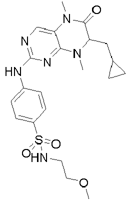 | 461.19 | 40 | 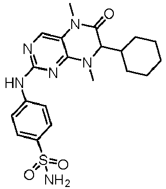 | 431.19 |
| 41 | 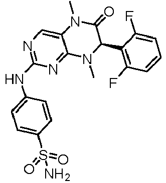 | 461.12 | 42 | 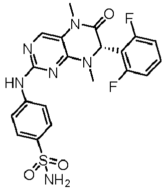 | 461.12 |
| 43 | 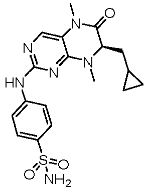 | 403.15 | 44 | 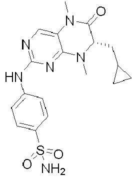 | 403.15 |
| 45 | 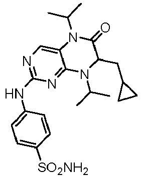 | 459.21 | 46 | 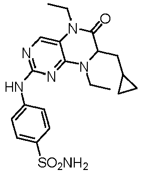 | 431.18 |
| 47 | 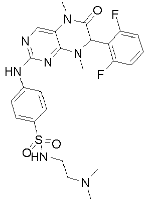 | 532.19 | 48 | 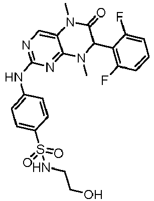 | 505.14 |
| 49 | 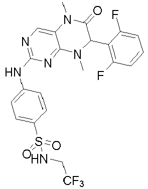 | 543.12 | 50 | 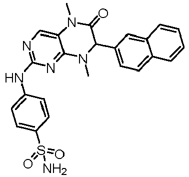 | 475.16 |
| 51 | 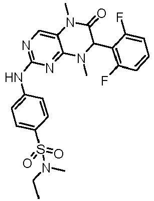 | 503.17 | 52 | 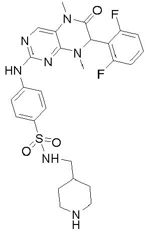 | 558.21 |
| 53 | 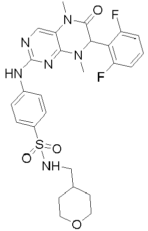 | 559.19 | 54 | 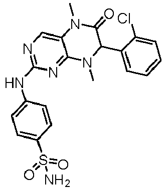 | 459.10 |
| 55 | 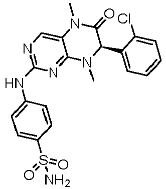 | 459.10 | 56 | 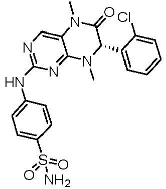 | 459.10 |
| 57 | 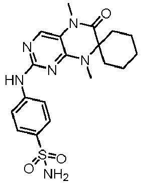 | 417.17 | 58 | 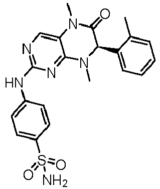 | 439.16 |
| 59 | 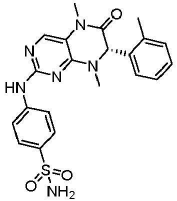 | 439.16 | | | |

### [Example 1]

### Isolation of human primary laryngeal cancer epithelial cells

Laryngeal cancer tissue samples were obtained from the cancer tissues of three informed and consenting laryngeal cancer patients by surgical resection, namely Sample Nos. 0S0020, 0S0021, and 0S0022. One of the samples (No. 0S0020) will be described below.

The aforementioned tissue samples were collected within half an hour after the surgical excision or the biopsy. More specifically, in a sterile environment, tissue samples from non-necrotic sites were cut with a volume of more than 0.5 cm³ and were placed in 4 mL of pre-cooled tissue transport fluid (specific formulation shown in Table 1). The transport fluid was placed in a 5 mL plastic sterile cryopreservation tube with a lid (purchased from Guangzhou Jet Bio-Filtration Co., Ltd.) and cold chain (0-10°C) transported to the laboratory.

**Tabel 1. Formulation of tissue transport fluid**

| Components of tissue transport fluid | Supplier | Final concentration |
|---|---|---|
| DMEM/F12 | Corning | 97.8 vol.% |
| Primocin | Invivogen | 0.2 vol.% (concentration of commercial product: 50 mg/ml) |
| penicillin / streptomycin solution double antibody | Corning | 2 vol.% (concentration of commercial product: 10000 U/ml penicillin, 10 mg/ml streptomycin) |

**Table 2. Fumulation of tissue digestive solution**

| Components of tissue digestive solution | Supplier | Final concentration |
|---|---|---|
| HBSS | Gibco | 50 vol.% |
| RPMI-1640 | Corning | 50 vol.% |
| collagenase II | Sigma | 2 mg/mL |
| collagenase IV | Sigma | 2 mg/mL |
| deoxyribonucleic acid I | Sigma | 50 U/mL |
| hyaluronidase | Sigma | 0.5 mg/mL |
| calcium chloride | Sangon Biotech | 0.33 mg/mL |
| bovine serum albumin | Sangon Biotech | 10 mg/mL |

In the biological safety cabinet, the tissue sample (No. 0S0020) was transferred to a 100 mm cell culture dish (purchased from NEST). The tissue sample was rinsed with the tissue transport fluid. The residual blood on the surface of the tissue sample was washed away. Excess tissues such as fat on the surface of the tissue sample were removed. The rinsed tissue sample was transferred to another new 100 mm culture dish; 2 mL of transport fluid was added, and a sterile scalpel blade and a forceps were used to divide the tissue sample into tissue fragments less than 3 mm³ in volume.

The tissue sample fragments were transferred to a 15 mL centrifuge tube, and centrifuged at 1500 rpm for 4 minutes in a tabletop centrifuge (Sigma, 3-18K); after discarding the supernatant, the tissue transport fluid and the tissue digestive solution were added in a ratio of 1:1 (the dosage is about 5 mL of tissue digestive solution per 10 mg of tissue; specific formulation was shown in Table 2); then the sample was numbered and sealed with sealing film, and was then digested in a constant-temperature shaker (Zhichu Instrument ZQLY-180N) at 37°C, 300 revolutions; whether the digestion was completed was determined via observation every 1 hour.

After digestion, undigested tissue blocks were filtered through a 70 µm filter screen; the tissue blocks on the filter screen were rinsed with the tissue transport fluid; the residual cells were rinsed into a centrifuge tube and centrifuged at 1500 rpm for 4 minutes.

After discarding the supernatant, the remaining cell clusters were observed to determine whether blood cells were remained; if there were blood cells, 3 mL blood cell lysate (purchased from Sigma) was added, which was then mixed well, lysed at 4°C for 15 minutes, with shaking and mixing well once every 5 minutes; after lysis, the resultant was take out and centrifuged at 1500 rpm for 4 minutes. The supernatant was discarded to provide digested and isolated primary laryngeal cancer cells, which were added with basic medium (BM) for resuspension. The basic medium was prepared by adding 0.2 vol.% of Primocin (purchased from Invivogen, with a concentration of 50 mg/mL) to the commercial DMEM/F-12 medium to provide a final concentration of 100 µg/mL. The total number of cells was 2,080,000, which was obtained by counting with a flow imaging counter (JIMBIO FIL, Jiangsu Jimbio Technology Co., Ltd.).

Other two laryngeal cancer tissue samples were isolated according to the same method above, and the total number of cells were 1,970,000 (0S0021) and 2,320,000 (0F0022), respectively.

### [Example 2]

### Optimization of culture medium for primary laryngeal cancer epithelial cell

### (1) Effects of different factors

The cultured NIH-3T3 cells (purchased from ATCC, and cultured in DMEM culture solution containing 10% fetal bovine serum) were digested with 0.25% trypsin (purchased from Thermo Fisher), and the digestion was terminated by using DMEM culture solution (purchased from Corning) containing 5%(v/v) fetal bovine serum (purchased from ExCell Biotech Co., Ltd.), 100 U/mL penicillin and 100 µg/mL streptomycin; the resultant was collected into a 15 mL centrifuge tube and centrifuged at 1500 rpm for 4 minutes, and then the supernatant was discarded. The centrifuged cell sediments were resuspended in the above DMEM culture solution containing 10% fetal bovine serum and were counted with a flow imaging counter (JIMBIO FIL, Jiangsu Jimbio Technology Co., Ltd.). The cells were irradiated with γ-ray of 35 Gy irradiation dose, and then were inoculated in a culture vessel at a density of 2×10⁴ cells/cm². The cells were incubated in a 37°C incubator until the cells adhere to the wall. Before the inoculation of primary cells, the culture medium was removed from the culture vessel.

Preparation of basic medium (abbreviated as BM): BM was prepared by adding 0.2 vol.% of Primocin (purchased from Invivogen, with a concentration of 50 mg/mL) to the commercial DMEM/F-12 medium to provide a final concentration of 100 µg/mL.

Next, different kinds and concentrations of additive factors (Table 3) were added to the basic medium (BM), so as to prepare culture mediums for laryngeal cancer epithelial cells containing different additive components.

**Table 3. Preparation of culture mediums containing different components (final concentrations are shown)**

| Culture medium | Sources of additive factors | Composition |
|---|---|---|
| Basic medium / BM | | DMEM/F12+100 µg/mL Primocin |
| BM + epidermal growth factor (EGF) | Sino Biological | BM + 80, 40, 20, 10, 5, 2.5, 1.25 ng/ml EGF |
| BM + hepatocyte growth factor (HGF) | Sino Biological | BM + 80, 40, 20, 10, 5, 2.5, 1.25 ng/ml HGF |
| BM + insulin-like growth factor 1 (IGF-1) | Sino Biological | BM + 80, 40, 20, 10, 5, 2.5, 1.25 ng/ml IGF-1 |
| BM + fibroblast growth factor (FGF) | Sino Biological | BM + 80, 40, 20, 10, 5, 2.5, 1.25 ng/ml FGF |
| BM + fibroblast growth factor 7 (FGF-7) | Sino Biological | BM + 80, 40, 20, 10, 5, 2.5, 1.25 ng/ml FGF-7 |
| BM + fibroblast growth factor 10 (FGF-10) | Sino Biological | BM + 80, 40, 20, 10, 5, 2.5, 1.25 ng/ml FGF-10 |
| BM + Neuregulin-1 | Sino Biological | BM + 80, 40, 20, 10, 5, 2.5, 1.25 ng/ml Neuregulin-1 |
| BM + Human Periostin | Sino Biological | BM + 80, 40, 20, 10, 5, 2.5, 1.25 ng/ml Human Periostin |
| BM + bovine pituitary extract | Sino Biological | BM + 80, 40, 20, 10, 5, 2.5, 1.25 ng/ml bovine pituitary extract |
| BM + N2 additive | Gibco | BM + 1/25, 1/50, 1/100, 1 /200, 1/400, 1/800, 1/1600 diluting ratio of N2 |
| BM + B27 additive | Gibco | BM + 1/25, 1/50, 1/100, 1 /200, 1/400, 1/800, 1/1600 diluting ratio of B27 |
| BM + insulin-transferrin-selenium complex (ITS) | Insulin, transferrin, sodium selenite all purchased from Sigma | BM + 1/25, 1/50, 1/100, 1 /200, 1/400, 1/800, 1/1600 diluting ratio of ITS stock solution (the stock solution contains 500 µg/ml insulin, 250 µg/ml transferrin, and 250 ng/ml sodium selenite in DMEM/F-12) |
| BM + non-essential amino acids | Gibco | BM + 1/25, 1/50, 1/100, 1 /200, 1/400, 1/800, 1/1600 diluting ratio of non-essential amino acids |
| BM + glutamine | Gibco | BM + 1/25, 1/50, 1/100, 1 /200, 1/400, 1/800, 1/1600 diluting ratio of glutamine |
| BM + Y27632 | MCE | BM + 40, 20, 10, 5, 2.5, 1.25, 0.625 µM Y27632 |
| BM + Compound 1 | Preparation Example | BM + 40, 20, 10, 5, 2.5, 1.25, 0.625 µM Compound 1 |
| BM + A83-01 | MCE | BM + 4000, 2000, 1000, 500, 250, 125, 62.5 nM A83-01 |
| BM + SB202190 | MCE | BM + 4000, 2000, 1000, 500, 250, 125, 62.5 nM SB202190 |
| BM + hydrocortisone | MCE | BM + 800, 400, 200, 100, 50, 25, 12.5 ng/ml hydrocortisone |
| BM + R-spondin1 | Sino Biological | BM + 800, 400, 200, 100, 50, 25, 12.5 ng/ml RSPO1 |
| BM + Noggin | Sino Biological | BM + 800, 400, 200, 100, 50, 25, 12.5 ng/ml Noggin |

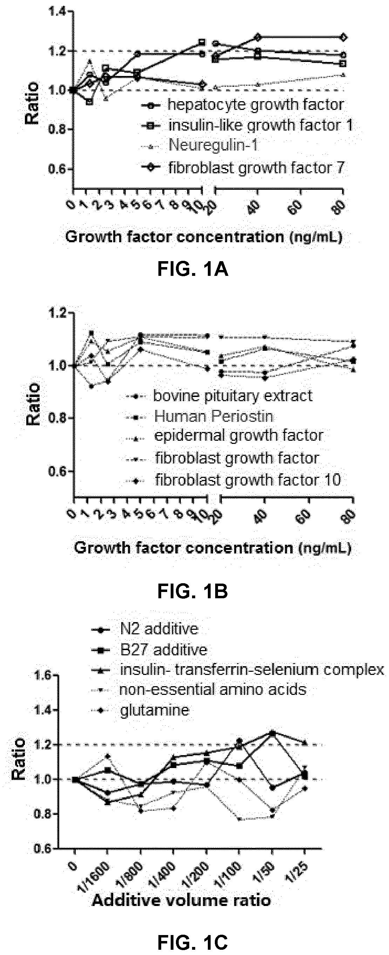

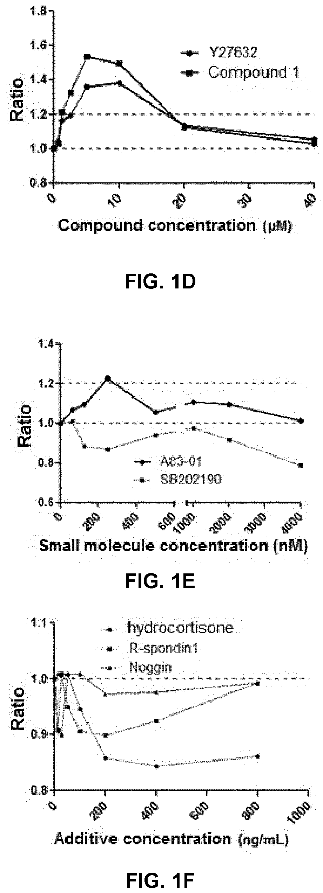

The culture mediums with different components were added at a volume of 500 µl/well to 48-well plates which were pre-laid with γ-ray irradiated NIH-3T3 cells. Laryngeal cancer cells (No. 0S0064) isolated from laryngeal cancer tissue according to the same method as described in Example 1 were inoculated at a cell count of 4×10⁴cells/well in the above-mentioned 48-well culture plates which were pre-laid with γ-ray irradiated NIH-3T3 cells. After surface disinfection, the plates were placed in a 37°C, 5% CO₂ incubator (purchased from Thermo Fisher), such that equal numbers of freshly isolated laryngeal cancer cells (No. 0S0064) were cultured under different medium formulations. The culture mediums were replaced and the trophoblastic cells were supplemented every 4 days. after the start of culture. After 10 days of culture, cell counts were performed. The basic medium (BM) without addition of any additive was used as the experimental control. The results were shown in Figs. 1A-1F.

The ordinate in the figures shows the ratio of the number of cells obtained after culture in different mediums to the number of cells obtained after culture in basic medium BM. As shown in the figures, adding different concentrations of different factors according to Table 3 to BM would result in different effects on cell proliferation. Among others, B27 additive, N2 additive, insulin-transferrin-selenium complex, hepatocyte growth factor, insulin-like growth factor 1, fibroblast growth factor 7, Compound 1, Y27632 and A83-01 provided certain promoting effects on cell proliferation in specific concentration ranges.

### (2) Effects of increasing factors in the culture mediums on the proliferation of primary laryngeal cancer cells obtained by the method of the invention

Different small molecules, additives and growth factors (Table 4) were sequentially added to the basic medium BM, respectively, so as to prepare culture mediums for laryngeal cancer epithelial cells containing different additive components.

**Table 4. Preparation of culture mediums containing different components (final concentrations are shown)**

| Medium | Composition |
|---|---|
| basic medium/BM | DMEM/F12+100 µg/mL Primocin |
| NO.1 | BM + 5 µM Compound 1 |
| NO.2 | NO.1 + 1:50 insulin- transferrin-selenium complex |
| NO.3 | NO.2 + 10 ng/mL hepatocyte growth factor |
| NO.4 | NO.3 + 10 µM Y27632 |
| NO.5 | NO.4 + 10 ng/mL insulin-like growth factor 1 |
| NO.6 | NO.5 + 40 ng/mL fibroblast growth factor 7 |
| NO.7 | NO.6 + 250 nM A83-01 |
| NO.8 | NO.7 + 10 ng/mL epidermal growth factor |
| NO.9 | NO.8 + 500 nM SB202190 |
| NO.10 | NO.9 + 10 µg/mL bovine pituitary extract |
| NO.11 | NO.10 + 1:50 B27 additive |
| NO.12 | NO.11 + 1: 100 N2 additive |
| NO.13 | NO.12 + 5% (volume ratio) fetal bovine serum |

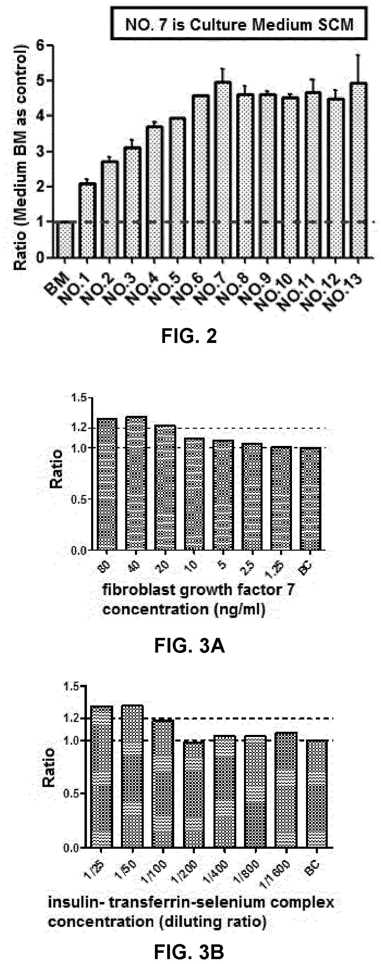

The culture mediums with different components were added at a volume of 500 µl/well to 48-well plates which were pre-laid with γ-ray irradiated NIH-3T3 cells, and simultaneously, the BM medium was used as the experiment control. Laryngeal cancer cells (No. 0S0065) isolated from laryngeal cancer tissue according to the method described in Example 1 were inoculated at a cell count of 4×10⁴ cells/well in the above-mentioned 48-well culture plates which were pre-laid with γ-ray irradiated NIH-3T3 cells. After surface disinfection, the plate was placed in a 37°C, 5% CO₂ incubator (purchased from Thermo Fisher), such that equal numbers of freshly isolated laryngeal cancer cells (No. 0S0065) were cultured under different medium formulations. After 7 days of culture, cell counts were performed. The results were shown in Fig. 2.

As shown in the figure, it was determined that No. 7 is the most preferred culture medium in this patent for culturing and expanding primary laryngeal cancer cells (hereinafter abbreviated as SCM). On this basis, further addition of some factors, small molecule inhibitors, or certain concentration of serum and substitute of serum, such as N2 additive and B27 additive, did not provide significant effect on promoting the cell proliferation.

### (3) Effects of different concentrations of the additive factors on the proliferation of primary laryngeal cancer cells obtained in the invention

Preparation of the culture medium for primary laryngeal cancer epithelial cells of the invention (abbreviated as SCM): to the basic medium (BM), was added fibroblast growth factor 7 (FGF-7) at the final concentration of 40 ng/ml, insulin-like growth factor 1 (IGF-1) at the final concentration of 10 ng/ml, hepatocyte growth factor (HGF) at the final concentration of 10 ng/ml, insulin-transferrin-selenium complex (ITS) stock solution at the diluting ratio of 1:50 (insulin at the final concentration of 10 µg/ml, transferrin at the final concentration of 5 µg/ml, and sodium selenite at the final concentration of 5 ng/ml in SCM medium), Compound 1 at the final concentration of 5 µM, Y27632 at the final concentration of 10 µM, and TGFβ1 inhibitor A83-01 at the final concentration of 250 nM, to prepare the culture medium for primary laryngeal cancer epithelial cells.

Laryngeal cancer epithelial cells originated from cancer tissue were isolated and obtained from the cancer tissue of a laryngeal cancer patient (Sample No. 0S0005) using the same method as in Example 1. Next, laryngeal cancer cells originated from cancer tissue were counted with a flow imaging counter (JIMBIO FIL, Jiangsu Jimbio Technology Co., Ltd.) to obtain the total number of cells. Then, the cells were inoculated in a 12-well plate which was pre-laid with γ-ray irradiated NIH-3T3 cells at a density of 4×10⁴ cells/cm². 2 mL of the prepared culture medium for primary laryngeal cancer epithelial cells SCM was added to the 12-well plate, which was then placed in a 37 °C, 5% CO₂ incubator (purchased from Thermo Fisher) for culture. When the cells in the culture plate grew to cover about 80% of the bottom area, the medium supernatant in the 12-well plate was discarded and 0.5 mL of 0.25% trypsin (purchased from Thermo Fisher) was added for digestion for 1 minute; after removing 0.25% trypsin, 0.5 mL of 0.05% trypsin was added again for cell digestion, which was then incubated at room temperature for 5-20 min, until the cells were completely digested as observed under the microscope (EVOS M500, Invitrogen); then the digestion was terminated by using 1 ml of DMEM/F12 culture solution containing 5% (v/v) fetal bovine serum, 100 U/mL penicillin, and 100 µg/mL penicillin; the resultant was collected into a 15 mL centrifuge tube and centrifuged at 1500 rpm for 4 minutes, and then the supernatant was discarded. The centrifuged cell sediments were resuspended in the basic medium BM and were counted with a flow imaging counter (JIMBIO FIL, Jiangsu Jimbio Technology Co., Ltd.) to obtain the total number of cells. The obtained cells were used in the following cultivation experiments.

Next, the following 7 culture mediums with different formulations were prepared for experiments:
Formulation 1: SCM medium composition without fibroblast growth factor 7;
Formulation 2: SCM medium composition without insulin-transferrin-selenium complex;
Formulation 3: SCM medium composition without insulin-like growth factor 1;
Formulation 4: SCM medium composition without hepatocyte growth factor;
Formulation 5: SCM medium composition without Y27632;
Formulation 6: SCM medium composition without Compound 1;
Formulation 7: SCM medium composition without A83-01.

The digested cell suspension was diluted with the above Formulations 1-7 respectively, and were seeded into a 48-well plate which was pre-laid with γ-ray irradiated NIH-3T3 cells with 10,000 cells and 250 µL volume per well.

When using the medium of Formulation 1, to a 48-well plate inoculated with primary cells was added the prepared fibroblast growth factor 7, with 250 µL per well, such that the final concentrations of fibroblast growth factor 7 were 80 ng/ml, 40 ng/ml, 20 ng/ml, 10 ng/ml, 5 ng/ml, 2.5 ng/ml, and 1.25 ng/ml, respectively; the medium of Formulation 1 was used as the control well (BC).

When using the medium of Formulation 2, to a 48-well plate inoculated with primary cells was added the prepared insulin-transferrin-selenium complex, with 250 µL per well, such that the final concentrations of insulin-transferrin-selenium complex stock solution were 1:1600, 1:800, 1:400, 1:200, 1:100, 1:50, and 1:25, respectively (corresponding to the final concentrations of insulin / transferrin / sodium selenite of 0.3125 µg/ml, 0.15625 µg/ml, 0.15625 ng/ml; 0.625 µg/ml, 0.3125 µg/ml, 0.3125 ng/ml; 1.25 µg/ml, 0.625 µg/ml, 0.625 ng/ml; 2.5 µg/ml, 1.25 µg/ml, 1.25 ng/ml; 5 µg/ml, 2.5 µg/ml, 2.5 ng/ml; 10 µg/ml, 5 µg/ml, 5 ng/ml; and 20 µg/ml, 10 µg/ml, 10 ng/ml, respecrtively); the medium of Formulation 2 was used as the control well (BC).

When using the medium of Formulation 3, to a 48-well plate inoculated with primary cells was added the prepared insulin-like growth factor 1, with 250 µL per well, such that the final concentrations of insulin-like growth factor 1 were 80 ng/ml, 40 ng/ml, 20 ng/ml, 10 ng/ml, 5 ng/ml, 2.5 ng/ml, and 1.25 ng/ml, respectively; the medium of Formulation 3 was used as the control well (BC).

When using the medium of Formulation 4, to a 48-well plate inoculated with primary cells was added the prepared hepatocyte growth factor, with 250 µL per well, such that the final concentrations of hepatocyte growth factor were 80 ng/ml, 40 ng/ml, 20 ng/ml, 10 ng/ml, 5 ng/ml, 2.5 ng/ml, and 1.25 ng/ml, respectively; the medium of Formulation 4 was used as the control well (BC).

When using the medium of Formulation 5, to a 48-well plate inoculated with primary cells was added the prepared Y27632, with 250 µL per well, such that the final concentrations of Y27632 were 40 µM, 20 µM, 10 µM, 5 µM, 2.5 µM, 1.25 µM, and 0.625 µM, respectively; the medium of Formulation 5 was used as the control well (BC).

When using the medium of Formulation 6, to a 48-well plate inoculated with primary cells was added the prepared Compound 1, with 250 µL per well, such that the final concentrations of Compound 1 were 40 µM, 20 µM, 10 µM, 5 µM, 2.5 µM, 1.25 µM, and 0.625 µM, respectively; the medium of Formulation 6 was used as the control well (BC).

When using the medium of Formulation 7, to a 48-well plate inoculated with primary cells was added the prepared A83-01, with 250 µL per well, such that the final concentrations of A83-01 were 4000 nM, 2000 nM, 1000 nM, 500 nM, 250 nM, 125 nM, and 62.5 nM, respectively; the medium of Formulation 7 was used as the control well (BC).

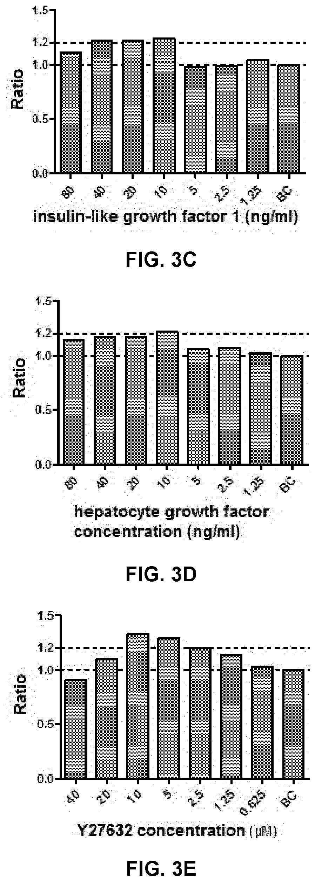

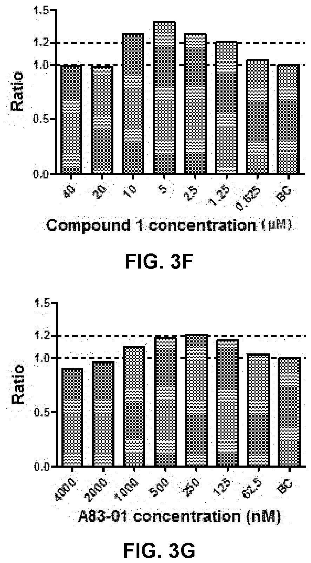

After the cells were expanded to about 85% of the 48 wells, the cells were digested and counted, the ratios were calculated by referring to the cell numbers in the control well (BC), and the results were shown in Figs. 3A-3G. In each of Figs. 3A-3G, the ratio represents a ratio of the number of cells of the first passage cultured by using each culture medium to the number of cells of the first passage cultured by the corresponding control well. If the ratio is greater than 1, it indicates that the proliferation promoting effect of the prepared medium containing different concentrations of factors or small molecular compounds is preferable over that of the control well medium; if the ratio is less than 1, it indicates that the proliferation promoting effect of the prepared medium containing different concentrations of factors or small molecular compounds is poorer than that of the control well medium.

According to the results of Figs. 3A -3G, the amount of fibroblast growth factor 7 in the culture medium is preferably 5 ng/ml-80 ng/ml, more preferably 20 ng/ml-80 ng/ml; the volume concentration of insulin-transferrin-selenium complex is preferably 1:25-1:100, more preferably 1:25-1:150 (corresponding to the final concentrations of insulin / transferrin / sodium selenite of 5-20 µg/ml, 2.5-10 µg/ml, 2.5-10 ng/ml, respectively, and more preferably 10-20 µg/ml, 5-10 µg/ml, 5-10 ng/ml, respectively); the amount of insulin-like growth factor 1 is preferably 10 ng/ml-80 ng/ml, more preferably 10 ng/ml-40 ng/ml; the amount of hepatocyte growth factor is preferably 10 ng/ml-80 ng/ml, more preferably 10 ng/ml-40 ng/ml; the amount of Y27632 is preferably 1.25 µM-20 µM, and more preferably 2.5 µM-10 µM; the amount of Compound 1 is preferably 1.25 µM-10 µM, and more preferably 2.5 µM-10 µM; the amount of A83-01 is preferably 125 nM-1000 nM, and more preferably 125 nM-500 nM.

### [Example 3]

### Culture of primary laryngeal cancer cells derived from human laryngeal cancer tissue

Laryngeal cancer epithelial cells originated from cancer tissue were isolated and obtained from the cancer tissue of a laryngeal cancer patient (Sample No. 0S0003) using the same method as in Example 1. Next, laryngeal cancer cells orginated from cancer tissue were counted with a flow imaging counter (JIMBIO FIL, Jiangsu Jimbio Technology Co., Ltd.) to obtain the total number of cells. Then, the cells were inoculated in a 12-well plate which was pre-laid with γ-ray irradiated NIH-3T3 cells at a density of 4×10⁴ cells/cm². 2 mL of the prepared culture medium for primary laryngeal cancer epithelial cells SCM was added to the 12-well plate, which was then placed in a 37 °C , 5% CO₂ incubator (purchased from Thermo Fisher) for culture.

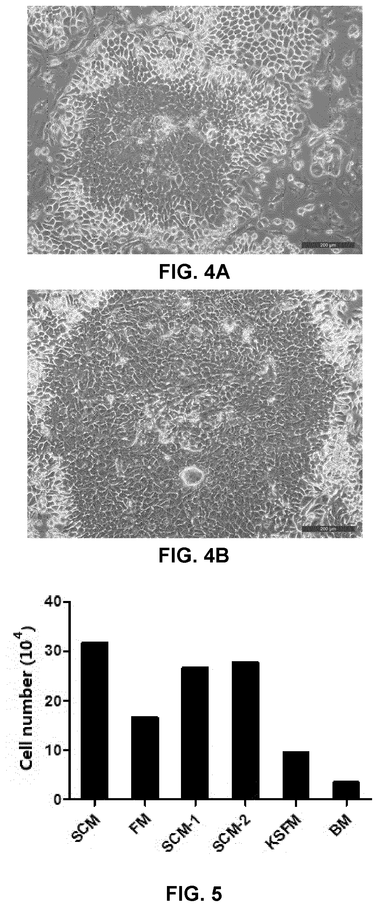

Fig. 4A is a microscopy image (photographed by a 100x inverted phase contrast microscope) of the cells which were cultured to day 4 after inoculation at a density of 4×10⁴ cells/cm² into a 12-well plate which was pre-laid with γ-ray irradiated NIH-3T3 cells according to this Example. Microscopic observation shows that the cultured primary laryngeal cancer cells derived from the cancer tissue had formed large clones. Fig. 4B is a microscopy image (photographed by a 100x inverted phase contrast microscope) of the cells which were cultured to day 12 after inoculation according to this Example, which shows that the field of vision was full of cells. It can be seen from Figs. 4A and 4B that the formation of obvious clone can be seen under microscope when the primary laryngeal cancer cells isolated were cultured *in vitro* for 4 days, and the number of cells was significantly expanded after 12 days of expansion, suggesting that the technology of the invention is a efficient technology for expanding laryngeal cancer epithelial cells *in vitro.*

### [Example 4]

### Effect of different culture mediums on promoting proliferation of laryngeal cancer tissue-derived primary laryngeal cancer cells

### (1) Comparison of the influences of different culture mediums on clone formation of primary cells and proliferation effects of primary cells

Culture medium for primary laryngeal cancer epithelial cell SCM was prepared in the same method of Example 2, and basic medium BM was prepared as control. As another control, a culture medium FM used in the cell conditional reprogramming technology was prepared additionally. For the preparation steps, please refer to Liu et al., Nat Protoc., 12(2): 439-451, 2017. The formulation of the culture medium is shown in Table 5. Simultaneously, as further controls, culture mediums for primary laryngeal cancer cell SCM-1 and SCM-2 were prepared, and the formulations of the culture mediums were obtained by replacing insulin-transferrin-selenium complex in medium SCM wth 1:50 volume ratio of B27 additive and 1:100 volume ratio of N2 additive, respectively. Moreover, as an additional control, a commercial medium Defined Keratinocyte SFM (hereinafter also referred to as "KSFM medium") was purchased from Gibco, and the formulation of the culture medium is shown in Table 6.

**Table 5. Composition of culture medium FM used in the cell conditional reprogramming technology**

| Medium composition | Supplier | Final concentration |
|---|---|---|
| DMEM medium | Corning | 65 vol.% |
| Fetal bovine serum | Gibico | 10 vol.% |
| Ham's F12 Nutrient Mixture | Gibico | 25 vol.% |
| Hydrocortisone | Sigma-Aldrich | 25 ng/ml |
| Epidermal growth factor | R&D | 0.125 ng/ml |
| Insulin | Sigma-Aldrich | 5 µg/ml |
| Amphotericin B | Sigma-Aldrich | 250 ng/ml |
| Gentamicin | Gibico | 10 µg/ml |
| Cholera Toxin | Sigma-Aldrich | 0.1 nM |
| Y27632 | Enzo | 10 µM |

**Table 6. Composition of commercial medium Defined Keratinocyte SFM (KSFM)**

| Medium composition | Supplier | Final concentration |
|---|---|---|
| Defined Keratinocyte-SFM Basal Medium | Gibco | 99 vol.% |
| Defined Keratinocyte-SFM Growth Supplement | Gibco | 1 vol.% |

By using the same method of Example 1, the primary laryngeal cancer cells (No. 0S0006) derived from laryngeal cancer tissues were obtained. Next, the cells were cultured at the same density (4×10⁴ cells/cm²) under the following 6 culture conditions:
A. The technology of the invention: the primary laryngeal cancer cells were inoculated into a 24-well plate which was pre-laid with γ-ray irradiated NIH-3T3 cells (purchased from ATCC) at a density of 4×10⁴ cells/cm², cultured using 1 mL of the culture medium for primary laryngeal cancer epithelial cells SCM of the invention;
B. Cell conditional reprogramming technology: the primary laryngeal cancer cells were inoculated into a 24-well plate which was pre-laid with γ-ray irradiated NIH-3T3 cells (purchased from ATCC) at a density of 4×10⁴ cells/cm², cultured in the 24-well plate using 1 mL of cell conditional reprogramming medium FM (the detailed steps, see Liu et al., Nat. Protoc., 12(2): 439-451, 2017);
C. The primary laryngeal cancer cells were inoculated into a 24-well plate which was pre-laid with γ-ray irradiated NIH-3T3 cells (purchased from ATCC) at a density of 4×10⁴ cells/cm², and cultured in the 24-well plate using 1 mL of the culture medium SCM-1;
D. The primary laryngeal cancer cells were inoculated into a 24-well plate which was pre-laid with γ-ray irradiated NIH-3T3 cells (purchased from ATCC) at a density of 4×10⁴ cells/cm², and cultured in the 24-well plate using 1 mL of the culture medium SCM-2;
E. The primary laryngeal cancer cells were inoculated into a 24-well plate at a density of 4×10⁴ cells/cm², and cultured in the 24-well plate using 1 mL of the commercial medium KSFM;
F. The primary laryngeal cancer cells were inoculated into a 24-well plate which was pre-laid with γ-ray irradiated NIH-3T3 cells (purchased from ATCC) at a density of 4×10⁴ cells/cm², and cultured in the 24-well plate using 2 mL of the basic medium BM.

In the above six cultures, the cells cultured under the six culture conditions wherein the mediums were renewed every 5 days. At the same time, the cell clone formation and the cell proliferation status under the cultivation of each medium in the 24-well plate was observed, and the cell growth status was recorded by taking pictures with a microscope (EVOS M500, Invitrogen).

To the primary cancer cells of laryngeal cancer (No. 0S0006) cultured with the technology of the invention, when the cells in the culture plate grew to cover about 80% of the bottom area, the medium supernatant in the 24-well plate was discarded and 0.5 mL of 0.25% trypsin (purchased from Thermo Fisher) was added for digestion for 1 minute; after removing 0.25% trypsin, 0.5 mL of 0.05% trypsin was added again for cell digestion, which was then incubated at 37°C for 10 minutes until the cells were completely digested as observed under the microscope (EVOS M500, Invitrogen); then the digestion was terminated by using 1 ml of DMEM/F12 culture solution containing 5% (v/v) fetal bovine serum, 100 U/mL penicillin, and 100 µg/mL penicillin; the resultant was collected into a 15 mL centrifuge tube and centrifuged at 1500 rpm for 4 minutes, and then the supernatant was discarded. The centrifuged cell sediments were resuspended in the culture medium of the invention and were counted with a flow imaging counter (JIMBIO FIL, Jiangsu Jimbio Technology Co., Ltd.) to obtain the total number of cells, which was 317,000. The cells cultured under the other five culture conditions were digested and counted in the same operation process as above. The total number of cells cultured by using the mediums FM, SCM-1, SCM-2, KSFM and BM were 166,700, 266,100, 277,200, 95,600 and 35,900, respectively.

Fig. 5 is a plot of the total number of cells amplified from No. 0S0006 cells under different conditions.

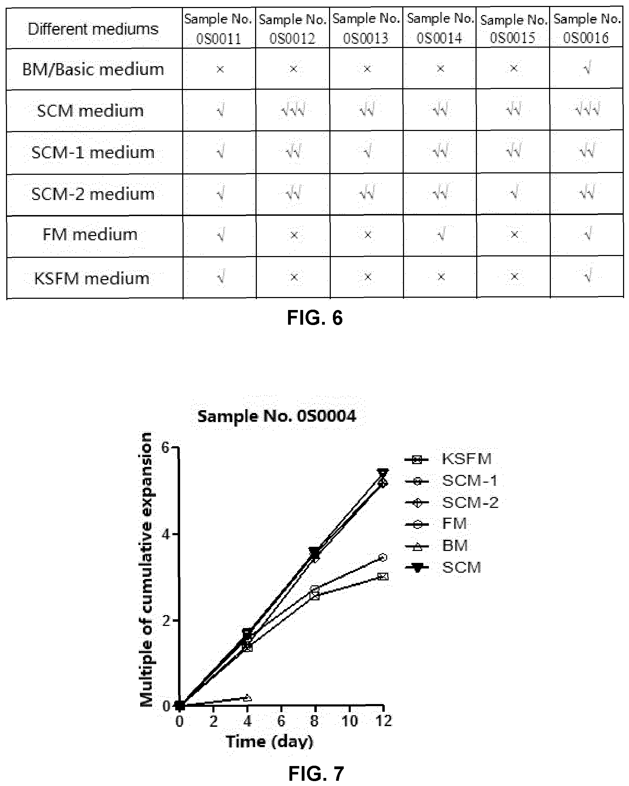

Fig. 6 is a comparative diagram of cell proliferation effects obtained by culturing the primary laryngeal cancer cells isolated from six laryngeal cancer patient samples (No. 0S0011, OS0012, OS0013, OS0014, OS0015, 0S0016) according to Example 1 for 7 days under the conditions of six different culture mediums, where √ represents a moderate clone formation ability and proliferation promoting effect, √ √ represents a significant clone formation ability and proliferation promoting effect, √ √ √ represents an extremely significant clone formation ability and proliferation promoting effect, and × represents no clone formation. It can be confirmed from the figure that the culture medium SCM has significant superiority over the other five culture conditions in terms of clone formation ability and cell proliferation promoting effect in cultivation of the laryngeal cancer tissue originated primary cell.

### (2) Continuous cultivation and growth curve of primary laryngeal cancer cells in different culture mediums

The culture medium for primary laryngeal cancer epithelial cell SCM and the culture mediums FM, SCM-1, SCM-2, KSFM and BM as controls were obtained by using the same method as in (1) of this Example.

The laryngeal cancer tissue originated primary laryngeal cancer cells (No. 0S0004) were cultured in six culture mediums, and then digested, passaged and counted by using the same method as in (1) of this Example.

When the passaged cells grew in the culture plate to cover about 80% of the bottom area of the plate again, the cultured cells were digested, collected and counted according to the above operating method. The cells were inoculated at a density of 4×10⁴ cells/well again and cultured continuously.

The following is the formula for calculating the cell population doubling number of primary laryngeal cancer epithelial cells under different culture conditions:
Population Doubling (PD) = 3.32 * log₁₀ (total number of digested cells / the number of cells at initial inoculation), see Chapman et al., Stem Cell Research & Therapy 2014, 5: 60.

Fig. 7 shows the growth curves of 0S0004 cells under six different culture conditions drawn by Graphpad Prism software. The abscissa represents the days of cell culture, and the ordinate represents the multiple of cumulative cell proliferation, i.e., the multiple of cell expansion in the culture period. The larger the value, the more multiples the cell expands in certain period, that is, the more cells are expanded. The slope represents the rate of cell expansion. It can be confirmed from the figure that the proliferation rates of laryngeal cancer epithelial cells cultured with the culture medium SCM of the invention and SCM-1, SCM-2 were superior to the other three culture conditions, and it can also be confirmed that the technology of the invention can continuously culture the primary laryngeal cancer epithelial cells.

### [Example 5]

### Identification of primary laryngeal cancer cells derived from cancer tissues

### (1) Immunofluorescence identification of primary laryngeal cancer tissues and laryngeal cancer cells after passage culture

Laryngeal cancer epithelial cells originated from cancer tissue were isolated and obtained from the cancer tissue of a laryngeal cancer patient (Sample No. 0S0015) using the same method as in Example 1. Next, laryngeal cancer cells originated from cancer tissue were counted with a flow imaging counter (JIMBIO FIL, Jiangsu Jimbio Technology Co., Ltd.) to obtain the total number of cells. Then, the cells were inoculated in a 24-well plate which was pre-laid with γ-ray irradiated NIH-3T3 cells at a density of 4×10⁴cells/cm². At the same time, round cell slides (purchased from Thermo Fisher) for immunofluorescence staining were pre-placed in the 24-well plate. 1 mL of the prepared culture medium for primary laryngeal cancer epithelial cells SCM was added to the 24-well plate, which was then placed in a 37°C, 5% CO₂ incubator (purchased from Thermo Fisher) for culture.

When the cells in the 24-well plate were expanded to cover 80% of the bottom area, the culture solution was discarded and 4% formaldehyde was used to fix the cells on ice for 30 minutes. The resultant was washed with PBS (purchased from Shanghai Sangon Biotech) for 5 minutes × 3 times. After discarding PBS, penetrating liquid was added, and the resultant was shaked (about 100 rpm) without exposure to light to break membrane for 30 minutes, and then was wash with PBS for 5 minutes × 3 times. Thereafter, PBS + 0.3% Triton X-100 (purchased from Shanghai Sangong Biotech) was used to prepare 5 vol.% BSA (purchased from Shanghai Sangong Biotech) solution for blocking, and the blocking was conducted at 37°C for 30 minutes.

PBS + 0.3% Triton X-100 was prepared in advance to dilute the antibody, wherein the squamous carcinoma specific antibody p63 (purchased from CST) was diluted in a ratio of 1:50. The blocking solution was discarded, and the prepared primary antibody diluent was added. The resultant was incubated in a refrigerator at 4°C overnight. The resultant was taken out at 4°C, balanced to room temperature, continued for incubation at 37°C for 1 hour, and then washed with PBS for 5 minutes × 3 times.

PBS + 0.3% Triton X-100 was prepared in advance for secondary antibody dilution, wherein the rabbit fluorescent secondary antibody (purchased from Thermo Fisher) with excitation light of 488 nm was diluted in a ratio of 1:1000. The resultant was incubated at room temperature in the dark for 1 hour, and then was washed with PBS for 5 minutes × 3 times.

Non-specific fluorescent dye DAPI (purchased from Sigma) was diluted with PBS in volume ratio of 1:1000, which was used for staining at room temperature in the dark for 5 minutes. The resultant was washed with PBS for 5 minutes × 3 times. Pictures were taken under microscope (EVOS M500, Invitrogen) for record.

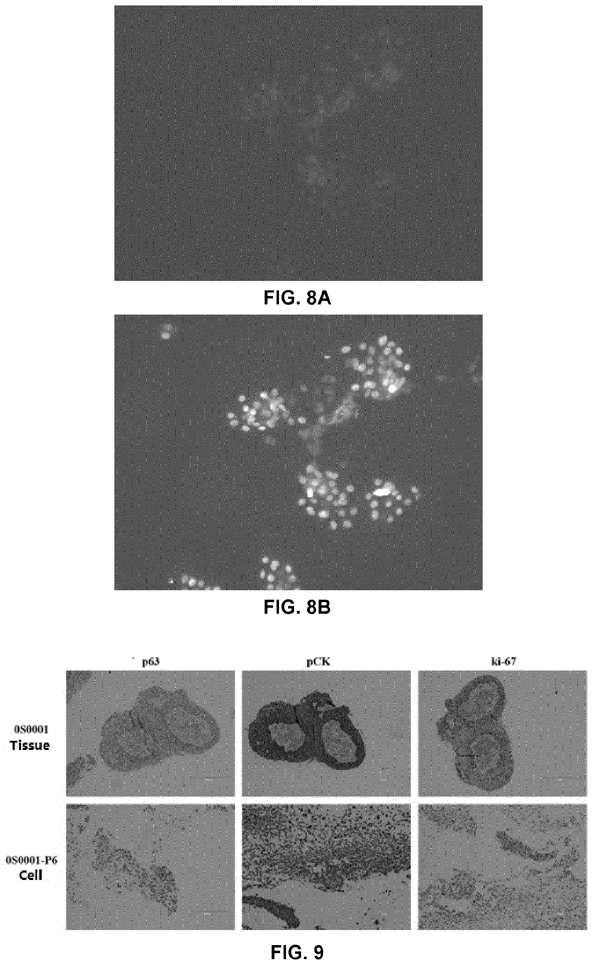

Figs. 8A and 8B show the fluorescent pictures taken in different fields under the 10X objective lens, respectively; in which, Fig. 8A is a picture of staining the nuclei with non-specific fluorescent dye DAPI, and Fig. 8B is a picture of staining with laryngeal cancer specific antibody p63 (located in the nuclei). As shown in the figures, the positions marked with nuclei in Fig. 8A were all stained green in Fig. 8B, indicating that the cultured cells are laryngeal squamous carcinoma cells, which are consistent with the clinical pathological diagnosis.

### (2) Immunohistochemical identification of primary laryngeal cancer tissues and laryngeal cancer cells after passage culture

Cancer tissue (Sample No. 0S0001) about the size of a mung bean was taken from a surgical resection sample of a laryngeal cancer patient, and immersed in 1 mL of 4% paraformaldehyde. Laryngeal cancer epithelial cells (Sample No. 0S0001) were obtained from the remaining cancer tissue in the same method of Example 1. Sample 0S0001 was continuously cultured to the sixth passage using the culture medium SCM of the invention according to the method of Example 3.

Immunohistochemistry assay was used to detect the expression of important laryngeal cancer-related biomarkers in the original tissue of Sample 0S0001 and the primary cells obtained by continuous culture to the sixth passage. The tissue was fixed with 4% paraformaldehyde, embedded in paraffin, and cut into tissue sections of 4 µm thickness with a microtome. Routine immunohistochemical detection was then performed (for detailed steps, see Li et al., Nature Communication, (2018) 9: 2983). The primary antibodies used were Cytokeratin (pCK) (purchased from CST), p63 antibody (purchased from CST), and Ki67 antibody (purchased from R&D).

Fig. 9 is a comparison of immunohistochemical results of the original tissue cells and the laryngeal cancer cells obtained by culturing the cells with the culture medium SCM of the invention. Fig. 9 confirms that the expression of laryngeal cancer-related biomarkers on the cells that were cultured from the laryngeal cancer cells (Sample No. 0S0001) by the technology of the invention to the 6th passage, was consistent with the expression of markers on the original tissue section from which the cells were derived. This suggests that the cells cultured by the technology of the invention maintain the original pathological characteristics of the cancer tissues of the laryngeal cancer patients.

### [Example 6]

### Xenograft tumorigenesis experiments of cancer tissue-derived primary laryngeal cancer cells in mice

Laryngeal cancer cells (No. 0S0003) were isolated and obtained from the cancer tissues of one pathologically diagnosed laryngeal cancer patient by using the same method as in Example 1. 0S0003 was cultured using the culture medium SCM of the invention according to the method of Example 3, and when the number of laryngeal cancer cells reached 1×10⁷, the laryngeal cancer cells were digested and collected by using the same method of Example 4. The culture medium for laryngeal cancer cells of the invention SCM and Matrigel^{®} (purchased from BD Biosciences) were mixed at a ratio of 1:1, and 100 µL of the culture medium mixed with Matrigel was used to resuspend 5×10⁶ laryngeal cancer cells, and the resultant was injected into the laryngeal cancer fat pad and the axilla of the right forelimb of 6-week-old female highly immunodeficient mice (NCG) (purchased from Nanjing Model Animal Research Center), respectively. The volume and growth rate of tumors in mice generated from the laryngeal cancer cells were observed and photographed every three days.

Tumor formation can be observed in both of the two tumor cell inoculation sites of the mice on day 21 after tumor cell inoculation. From day 21 to day 40, the tumor proliferation in mice was obvious. This indicates that the cancer tissue-derived laryngeal cancer cells cultured by the culture method of the invention have tumorigenicity in mice.

### [Example 7]

Drug sensitivity functional test of cancer tissue-derived laryngeal cancer cells

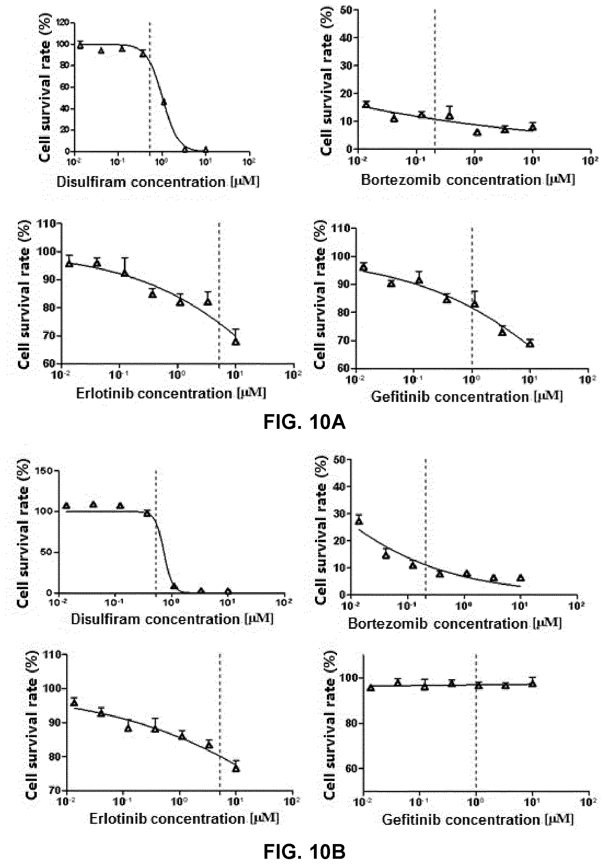

Taking a surgical resection sample from a laryngeal cancer patient as an example, it is verified that the laryngeal cancer cells cultured from the patient-derived laryngeal tumor samples can be used to test the sensitivity of the tumor cells of the patient to different drugs.
1. Plating of primary laryngeal cancer cells: Cell suspensions of isolated laryngeal cancer cells (No. 0S0020 and No. 0S0022) obtained according to the method of Example 1, were inoculated in a 12-well plate which was pre-laid with γ-ray irradiated NIH-3T3 cells at a density of 4×10⁴ cells/cm². 2 mL of the prepared culture medium for primary laryngeal cancer epithelial cells SCM was added to the 12-well plate, which was then placed in a 37°C, 5% CO₂ incubator (purchased from Thermo Fisher) for culture. When the cells in the culture plate grew to cover about 80% of the bottom area, the medium supernatant in the 12-well plate was discarded and 0.5 mL of 0.25% trypsin (purchased from Thermo Fisher) was added for digestion for 1 minute; after removing 0.25% trypsin, 0.5 mL of 0.05% trypsin was added again for cell digestion; the cells were incubated at 37°C for 10 minutes until the cells were completely digested as observed under the microscope (EVOS M500, Invitrogen); then the digestion was terminated by using 1 mL of DMEM/F12 culture solution containing 5% (v/v) fetal bovine serum, 100 U/mL penicillin, and 100 µg/mL penicillin; the resultant was collected into a 15 mL centrifuge tube and centrifuged at 1500 rpm for 4 minutes, and then the supernatant was discarded. The centrifuged cell sediments were resuspended in the culture medium SCM and were counted with a flow imaging counter (JIMBIO FIL, Jiangsu Jimbio Technology Co., Ltd.) to obtain the total number of cells, which were 830,000 and 768,000, respectively. The cells were incubated into a 384-well plate at a density of 2,000-4,000 cells/well, and the cells were adhered overnight.
2. Drug gradient experiments:
   (1) The drug storage plates were prepared by gradient dilution method: 40 µL of 10 µM drug stock solutions to be tested were respectively taken as the maximum concentrations, and 10 µL of solutions were respectively pipetted therefrom and added into 0.5 mL EP tubes containing 20 µL of DMSO; 10 µL of solutions from the above EP tubes were pipetted again into second 0.5 mL EP tubes loaded with 20 µL of DMSO, that is, diluting the drugs in a ratio of 1:3. The above method was repeated for gradually dilution and 7 concentrations required for dosing were obtained. Different concentrations of drugs were added to the 384-well drug storage plates. Equal volume of DMSO was added to each well of the solvent control group as a control. In this example, the drugs to be tested were Bortezomib (purchased from MCE), Disulfiram (purchased from MCE), Gefitinib (purchased from MCE), and Erlotinib (purchased from MCE).
   (2) Using a high-throughput automated workstation (JANUS, Perkin Elmer), different concentrations of drugs and solvent controls in the 384-well drug storage plates were added to 384-well cell culture plates plated with the laryngeal cancer cells. The drug groups and the solvent control groups were each arranged with 3 duplicate wells. The volume of drugs added to each well was 100 nL.
   (3) Test of cell viability: 72 hours after administration, Cell Titer-Glo assay kit (purchased from Promega) was used to detect the chemiluminescence value of the cultured cells after drug administration. The magnitude of the chemiluminescence value reflects the cell viability and the effect of the drug on the cell viability. 10 µL of the prepared Cell Titer-Glo detection solution was added to each well, and a microplate reader (Envision, Perkin Elmer) was used to detect the chemiluminescence value after mixing.
   (4) Test of cell viability: According to the formula, Cell survival rate (%) = Chemiluminescence value of drug well / Chemiluminescence value of control well *100%, the cell survival rates of cells treated with different drugs were calculated. Graphs were made by using Graphpad Prism software and the half-inhibition rates IC₅₀ were calculated, and simultaneously, the cell survival rates under the maximum blood concentrations Cₘₐₓ of different drugs in human body were calculated.
   (5) The drug sensitivity testing results were shown in Fig. 10.

Figs. 10A and 10B respectively represent the sensitivity of the laryngeal cancer cells cultured from surgically resected cancer tissue samples of two different laryngeal cancer patients (Sample No. 0S0020 and Sample No. 0S0022) to two chemotherapeutic drugs Bortezomib and Disulfiram, and to targeted drugs Gefitinib and Erlotinib. Specifically, Fig. 10A shows the sensitivity results of laryngeal cancer cells cultured from Sample No. 0S0020 on four drugs; Fig. 14B shows the sensitivity results of laryngeal cancer cells cultured from Sample No. No. 0S0022 on four drugs. In the figures, the concentrations on the abscissa corresponding to the dash lines are the maximum blood concentrations Cₘₐₓ of these four drugs in human body. The results show that the cells from the same patient have different sensitivities to different drugs at their respective maximum blood concentrations in human body, and the cells from different patients also have different sensitivities to the drugs at the maximum blood concentrations in human body. According to the results, the effectiveness of the drugs in the clinical use to the laryngeal cancer patients can be determined.

### Industrial applicability

The invention provides a culture medium and a culture method for culturing or expanding primary laryngeal cancer epithelial cells *in vitro.* The cell progeny and organoid cultured using the culture medium and culture method of the invention can be used for the efficacy evaluation and screening of drugs, toxicity determination, and regenerative medicine. Therefore, the invention is applicable in the industry.

Although the present invention has been described in details herein, the present invention is not limited thereto, and those skilled in the art can make modification according to the principle of the invention. Therefore, all modifications made according to the principle of the invention should be interpreted as falling within the protection scope of the invention.

## Claims

1. A primary cell culture medium for culturing laryngeal cancer epithelial cells, **characterized in that**:
comprising an MST1/2 kinase inhibitor, wherein the MST1/2 kinase inhibitor comprises a compound of Formula (I) or a pharmaceutically acceptable salt, or a solvate thereof, 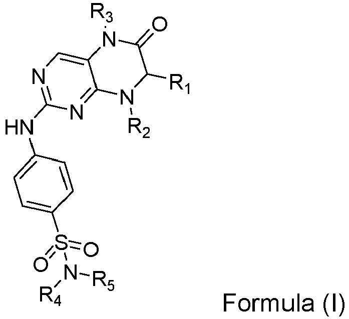 wherein,
R₁ is selected from C1-C6 alkyl, C3-C6 cycloalkyl, C4-C8 cycloalkylalkyl, C2-C6 spirocycloalkyl, and aryl optionally independently substituted with 1-2 R₆, aryl C1-C6 alkyl optionally independently substituted with 1-2 R₆ and heteroaryl optionally independently substituted with 1-2 R₆;
R₂ and R₃ are each independently selected from C1-C6 alkyl;
R₄ and R₅ are each independently selected from hydrogen, C1-C6 alkyl, C3-C6 cycloalkyl, C4-C8 cycloalkylalkyl, hydroxyl C1-C6 alkyl, C1-C6 haloalkyl, C1-C6 alkylamino C1-C6 alkyl, C1-C6 alkoxy C1-C6 alkyl, and C3-C6 heterocyclyl C1-C6 alkyl;
R₆ is selected from halogen, C1-C6 alkyl, C1-C6 alkoxy, and C1-C6 haloalkyl.

2. The primary cell culture medium of claim 1, wherein,
R₁ is selected from C1-C6 alkyl, C3-C6 cycloalkyl, C4-C8 cycloalkylalkyl, C2-C6 spirocycloalkyl, and phenyl optionally independently substituted with 1-2 R₆, naphthyl optionally independently substituted with 1-2 R₆, phenylmethyl optionally independently substituted with 1-2 R₆ and thienyl optionally independently substituted with 1-2 R₆;
R₂ and R₃ are each independently selected from C1-C3 alkyl;
R₄ and R₅ are each independently selected from hydrogen, C1-C6 alkyl, C3-C6 cycloalkyl, C4-C8 cycloalkylalkyl, hydroxyl C1-C6 alkyl, C1-C6 haloalkyl, C1-C6 alkylamino C1-C6 alkyl, C1-C6 alkoxy C1-C6 alkyl, piperidyl C1-C6 alkyl, and tetrahydropyranyl C1-C6 alkyl;
R₆ is selected from halogen, C1-C6 alkyl, C1-C6 alkoxy, and C1-C6 haloalkyl.

3. The primary cell culture medium of claim 1, wherein the MST1/2 kinase inhibitor comprises a compound of Formula (Ia) or a pharmaceutically acceptable salt, or a solvate thereof, 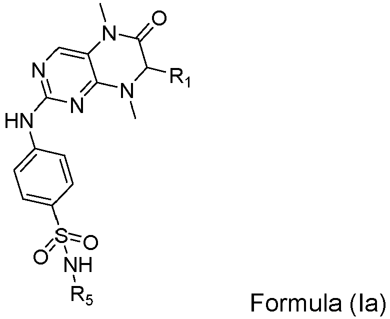 wherein,
R₁ is selected from C1-C6 alkyl, phenyl optionally independently substituted with 1-2 R₆, thienyl optionally independently substituted with 1-2 R₆, and phenylmethyl optionally independently substituted with 1-2 R₆;
R₅ is selected from hydrogen, C1-C6 alkyl, and C3-C6 cycloalkyl;
R₆ is independently selected from halogen, C1-C6 alkyl, and C1-C6 haloalkyl.

4. The primary cell culture medium of claim 3, wherein
R₁ is phenyl optionally independently substituted with 1-2 R₆;
R₅ is hydrogen;
R₆ is preferably fluoro, methyl or trifluoromethyl.

5. The primary cell culture medium of claim 1, wherein the MST1/2 kinase inhibitor is at least one selected from the following compounds or a pharmaceutically acceptable salt thereof:
| | | | |
|---|---|---|---|
| 1 | 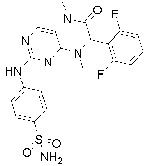 | 2 | 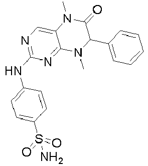 |
| 3 | 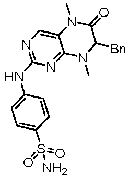 | 4 | 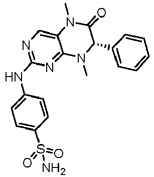 |
| 5 | 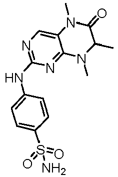 | 6 | 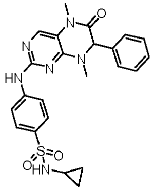 |
| 7 | 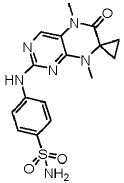 | 8 | 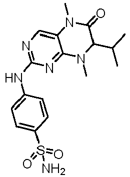 |
| 9 | 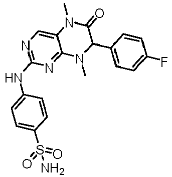 | 10 | 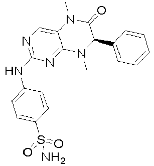 |
| 11 | 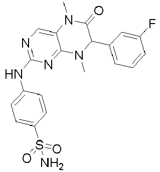 | 12 | 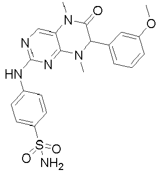 |
| 13 | 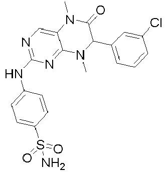 | 14 | 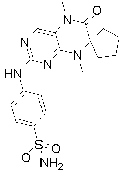 |
| 15 | 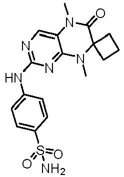 | 16 | 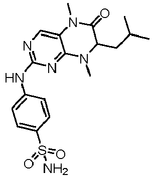 |
| 17 | 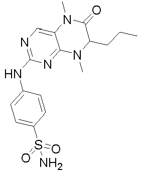 | 18 | 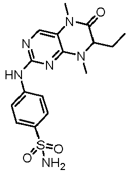 |
| 19 | 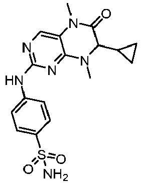 | 20 | 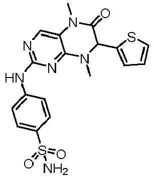 |
| 21 | 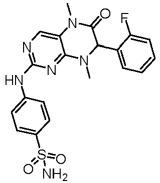 | 22 | 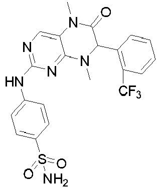 |
| 23 | 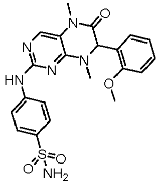 | 24 | 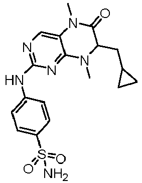 |
| 25 | 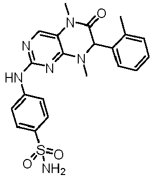 | 26 | 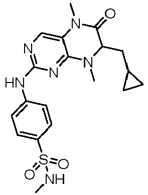 |
| 27 | 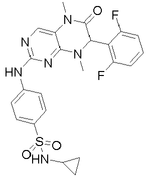 | 28 | 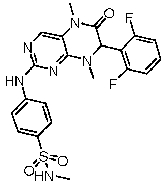 |
| 29 | 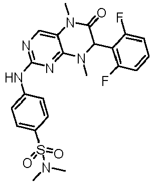 | 30 | 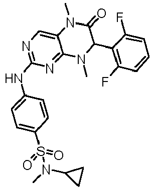 |
| 31 | 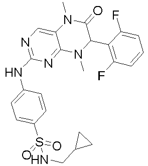 | 32 | 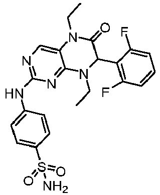 |
| 33 | 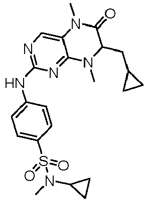 | 34 | 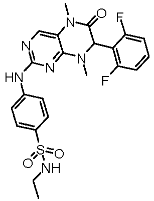 |
| 35 | 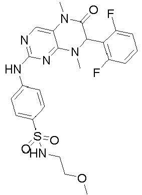 | 36 | 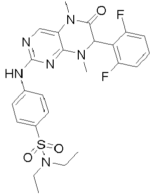 |
| 37 | 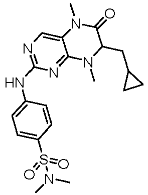 | 38 | 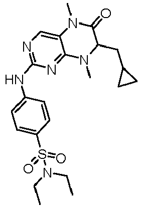 |
| 39 | 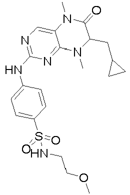 | 40 | 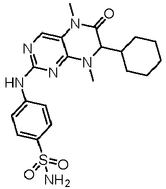 |
| 41 | 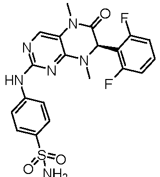 | 42 | 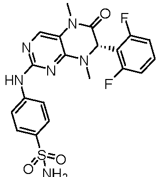 |
| 43 | 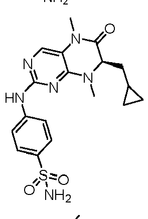 | 44 | 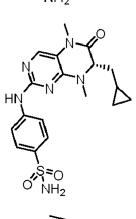 |
| 45 | 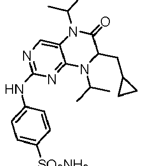 | 46 | 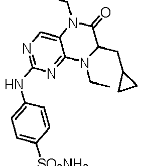 |
| 47 | 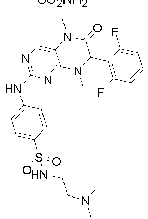 | 48 | 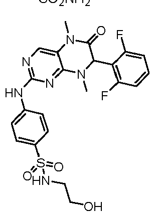 |
| 49 | 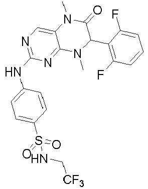 | 50 | 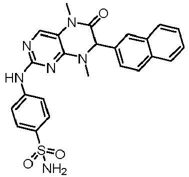 |
| 51 | 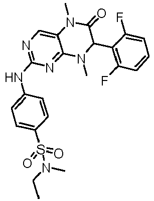 | 52 | 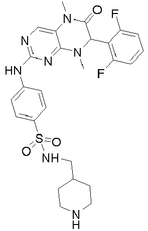 |
| 53 | 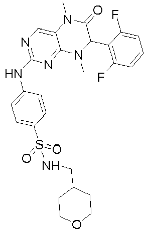 | 54 | 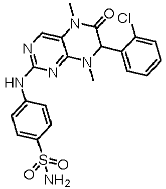 |
| 55 | 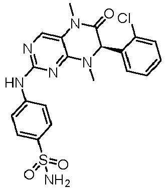 | 56 | 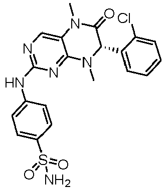 |
| 57 | 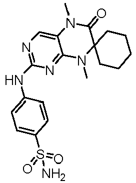 | 58 | 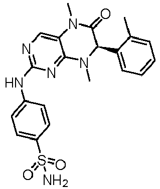 |
| 59 | 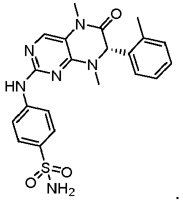 | | |

6. The primary cell culture medium of any one of claims 1-5, **characterized in that**:
the amount of the MST1/2 kinase inhibitor is 1.25-10 µM, preferably 2.5-10 µM.

7. The primary cell culture medium of any one of claims 1-5, **characterized in that**:
further containing one or more of the following factors: insulin-like growth factor 1; fibroblast growth factor 7; insulin-transferrin-selenium complex; hepatocyte growth factor; a ROCK kinase inhibitor selected from at least one of Y27632, Fasudil, and H-1152; and a TGFβ type I receptor inhibitor selected from at least one of A83-01, SB431542, Repsox, SB505124, SB525334, SD208, LY36494, and SJN2511.

8. The primary cell culture medium of claim 7, **characterized in that**:
the amount of insulin-like growth factor 1 is 10-80 ng/ml, more preferably 10-40 ng/ml;
the amount of fibroblast growth factor 7 is 5-80 ng/ml, more preferably 20-80 ng/ml;
the respective amounts of insulin / transferrin / sodium selenite in the insulin-transferrin-selenium complex are 5-20 µg/ml, 2.5-10 µg/ml, 2.5-10 ng/ml, respectively, and more preferably 10-20 µg/ml, 5-10 µg/ml, 5-10 ng/ml, respectively;
the amount of hepatocyte growth factor is 10-80 ng/ml, more preferably 10-40 ng/ml;
the amount of the ROCK kinase inhibitor is 1.25-20 µM, more preferably 2.5-10 µM);
the amount of the TGFβ type I receptor inhibitor is 125 -1000 nM, more preferably 125-500 nM.

9. The primary cell culture medium of any one of claims 1-5, **characterized in that**:
being free of serum, bovine pituitary extract, Wnt agonists, R-spondin family proteins, BMP inhibitors, nicotinamide, or N-acetylcysteine.

10. The primary cell culture medium of any one of claims 1-5, **characterized in that**:
the laryngeal cancer epithelial cells are selected from laryngeal cancer cells, normal laryngeal cancer epithelial cells, and laryngeal cancer epithelial stem cells.

11. A method for culturing laryngeal cancer epithelial cells, **characterized in** comprising the following steps:
(1) preparing the primary cell culture medium according to any one of claims 1-10;
(2) pre-laying a culture vessel with trophoblastic cells irradiated with X-ray or γ-ray_{;}
(3) inoculating primary laryngeal cancer epithelial cells isolated from laryngeal cancer tissues in the culture vessel which is pre-laid with trophoblastic cells, and culturing by using the primary cell culture medium of step (1).

12. A method for evaluating or screening a drug for treating laryngeal cancer diseases, **characterized in that**, comprising the following steps:
(1) culturing laryngeal cancer epithelial cells by the culturing method of claim 11;
(2) selecting the drug to be tested and diluting into different drug concentration gradients;
(3) adding the drug which has diluted to gradients to the laryngeal cancer epithelial cells obtained in step (1), and detecting the cell viability.
